# EUROPEAN PATENT APPLICATION

(11) **EP 4 847 596 A1**
(43) Date of publication of application: **02.12.2026**
(21) Application number: 25305755.8
(22) Date of filing: 26.05.2025
(51) Int. Cl.: C12M 1/00

(54) **FLOW-THROUGH FLUID FILTERING ASSEMBLY FOR BIOPHARMACEUTICAL PRODUCT, USE OF THE FILTERING ASSEMBLY FOR VISIBLE PARTICLES RETENTION, AND METHOD TO OBTAIN A BAG UNIT PROVIDED WITH THE FILTERING ASSEMBLY**

(71) Applicant: Sartorius Stedim FMT, 13400 Aubagne (FR)
(72) Inventor: GAY, Isabelle, 13400 Aubagne (FR)
(74) Representative: Plasseraud IP

(57) **Abstract**

An inlet or outlet, formed by a tubular male member (4a) for attachment to a flexible hose (T), is housing a filter device (5) whose trap portion (8) is embedded internally in a hollow fluid coupler. A flowable biopharmaceutical product filtering assembly (1) is formed with the filer device (5) maintained in an inserted position in lumen (40) of the male member when an axially permeable plugging part or head (6) is engaged in the member conduit (SC) to axially maintain the trap portion (8) in the inserted position. The filter device (5) can be inserted along an insertion direction with a filter insertion end (7) firstly inserted in lumen (40) and then locked with the elongated trap portion (8), disposed adjacent to the plugging part and configured to purify the flowable product by keeping the visible solid particles having a size superior to 100 micrometres downstream the plugging part.

## Description

### Technical Field

This disclosure pertains to the field of fluid transfer in biopharmaceutical field, for instance at a final stage before use of drug products or similar biopharmaceutical compositions that are liquid/flowable, when using filters (typically filters of the IPC class B01D27) to retain solid particles. More precisely, a fluid filtering assembly with embedded filter device is provided to retain visible particles and a method using this assembly for calibrating a single-use sensor is also proposed.

### Background Art

In biotechnology processes, final drug products or similar content are carefully analyzed and cannot be polluted. Many control steps, separation stage can be involved at the time of preparing the biopharmaceutical composition, typically in a bioreactor. Sterilization and/or purifying steps are performed until obtaining a final flowable/liquid product. Document WO8101248A1 shows an exemplary process for transferring a parenteral solution into an extensible plastic pouch. A filter is generally interposed between the solution reservoir(s) and the bag unit including the pouch.

Of course, the products of interest to be retrieved after filtration are not water or a liquid solvent; this is why the term "biopharmaceutical" is consistently used in the instant disclosure. Filtration part must be configured to prevent retention of the valuable biopharmaceutical compounds of the biopharmaceutical product. The wording "flowable biopharmaceutical product" is thus to be understood as meaning a product (distinct from a drink or water), flowable/liquid, coming from biotechnology, culture mediums, cell cultures, buffer solutions, artificial nutrition liquids, blood products and derivatives of blood products, or a pharmaceutical product.

To facilitate assembling steps, it is known using in-line filters. For instance, filtration devices (of the type Sartopore^{®} or the like) can provide a high level of filtration, with a Polyethersulfone membrane interposed between two nozzles having each a hose barb design for connection to a flexible hose. Additional sterilizing steps may be involved due to the connections of such filter. When having a parenteral solution or the like obtained in biotechnology processes, to be transferred, late risk of contamination can be an issue. Flushing single use systems could be a solution, but it brings complexity to the design, the assembling process and the steps to be controlled.

In the biopharmaceutical industry, there is still a need to:
- improve the process of transferring fluids properly in the circulation paths involving single-use equipment or similar containing parts frequently used in this technical field;
- reduce the amount of material involved to limit the disposal of components (plastic components);
- and retrieve the valuable molecules, biomedical compounds, cells, what is here called the flowable biopharmaceutical composition/product, at a given location, possibly final location before their use (biomedical use for instance) without allowing impurities or contaminants to be present.

Practically, if undesirable particles are detected, the valuable biopharmaceutical product can be rejected, thus disposed (due to strong safety concern), which raises significant economic losses. Some large particles (those visible by the human eye) may also appear at any time in a bioprocess, for instance due to the way the plastic single-use are manufactured or handled/assembled.

More generally, there is still room for improvement regarding constructions of fluid transfer systems to allow proper collection of biopharmaceutical product.

### Objects and Summary

For improving situation, a filter device/unit capable or separating a range of particles that are visible is provided for a proper integration in a segment of conduit, typically formed by a tubular male member for attachment to a flexible hose, in a fluid connection interface. Embodiments of the invention provide a flowable biopharmaceutical product (liquid product typically) filtering assembly, comprising:
- a hollow fluid connection interface having two opposite ends and allowing circulation of the product;
- a filter device disposed within the hollow fluid connection interface, in an inserted position; wherein the hollow fluid connection interface includes a fluidic connection member, forming/including a male tubular member to have a segment of conduit defining a longitudinal lumen through which the flowable biopharmaceutical product can pass;
wherein the filter device is configured to selectively retain visible solid particles which have a size superior or equal to 100 micrometres, the filter device comprising:
- a plugging part that is permeable;
- a filter insertion end; and
- a trap portion that is elongated, along a longitudinal axis of the segment of conduit (for instance elongated to be preferably longer than 3 or 6 mm), while being disposed adjacent to the plugging part and configured to purify the flowable biopharmaceutical product by separating and keeping the visible solid particles downstream the plugging part, between the plugging part and the filter insertion end provided opposite from the plugging part;
and wherein the trap portion extends inside the lumen and is axially locked or maintained in the inserted position by the plugging part.

Thanks to this arrangement, a quite simple filtration not impairing the liquid circulation/rate (with low or minor pressure drop) is obtained, possibly in a simple male connector having a segment of conduit (having a longitudinal axis) extending longitudinally:
- between a free end of the tubular male member, which is an elongated connecting member (possibly of a standard construction with a narrow passage/lumen inner diameter, lower than 20 or 25 mm typically) that is configured for attachment to flexible hose;
- and a mouth, which is preferably an axial mouth.

With such embodiments the filter device is integrated in compact manner, with the trap portion and the male member for hose connection sharing a common volume involved for circulation of the biopharmaceutical fluid/product.
The fluid connection interface may be a rigid coupler, so that the fluidic connection member is rigid, typically being formed by a single piece/monolithic part. It is obtained a connection and liquid separation component, typically with fine mesh, beads or 3D porous structure designed to retain visible particles (≥ 100µm) from a flowing liquid solution, while keeping a robust/easy way to perform the fluid connection, in a sterile manner for instance.

Thanks to this arrangement, less waste of valuable biopharmaceutical product is allowed, without involving an additional bulk in the circuit or path. The lumen can be used to form a filtration chamber in a narrow space, with no need to structurally modify/transform the tubular male member of the interface, without any enlargement in conception of the fluid transfer parts.

The plugging part is typically an axially permeable plugging part, for instance provided with an annular part that is not permeable (without any side wall aperture) and/or with an annular sealing element surrounding an axial access of the plugging part. A transverse wall, permeable to allow the liquid to flow through, may optionally extend in the plugging part, with such transverse wall being formed through an opening or central hollow of the filter device that defines a fluid filter inlet. Alternatively, a porous head or enlarged end of the filtering device can be provided to allow passage of the liquid product, with a porosity level in the head/enlarged end that has a lower filtration level than a filtration level provided by the portion.

The plugging part may allow a removable connection inside the segment of conduit, which means separation may be easy after use, for instance for recycling or re-using the fluid connection interface and selectively disposing the filter device.

The filter device has a tubular outer surface with the trap portion defining a maximum diameter lower than 20 or 25 mm. The corresponding lumen for receiving the filter device may may be not wider than 29 mm, possibly not wider than 16 or 24 mm. The section of the lumen may be constant or slightly vary. When the segment of conduit has a cross section that varies, it is understood the lumen/lumen area where the trap portion extends can be provided with narrow/narrowest section of the piece forming the fluid connection member. The trap portion is preferably surrounded by an inner face of the tubular male member, due to the fact that the plugging part is shorter than the trap portion and/or the inserted section of the plugging part in the lumen is shorter than the trap portion. The plugging part can be at least three times shorter than the trap portion.

The filter device can be made of one or more materials, possibly only including biocompatible material. For instance, the filter device can be made of a piece of plastic (thermoplastics), ceramic, metal. A tubular grid or mesh can be formed to delimit an outer face of the trap portion, with the tubular grid surrounding a filtration chamber area where the visible particles are retained.

The filter device can be constructed with a compact and elongated structure to have a short plugging part, possibly provided with a lip or collar allowing axial abutment on a surround of an axial access formed at free end of the male member.
In embodiments, the plugging part has a central passage, preferably closed by a grid or permeable radial/transverse portion, the central passage extending from an open axial free end to a central access defining an inlet of the trap portion.
The plugging part can have a cavity delimited by a radial/transverse grid, the radial grid separating the cavity from a central hollow of the trap portion.

The filter device is adapted to be mounted inside the lumen to be surrounded by the hose in a hose attachment configuration. In embodiments of the filtering assembly that allows such attachment configuration, one or more of the following features are provided:
- a barb fitting is provided in the fluidic connection member to form the male member, so that at least one barb surrounds the filter device.
- the barb fitting (or similar fitting) is providing a needleless access/passage (no needle being present).
- the barb fitting may be integral with a plastic bag used in the pharmaceutical and biopharmaceutical industry.
- a flange, forming an enlarged portion of the fluid connection interface, is provided at the opposite from the barb of the barb fitting.
- the tubular male member comprises an outer annular relief or barb and is configured for attachment to the flexible hose by being inserted inside a hose end of the flexible hose.

Embodiments of the filtering assembly provide one or more of the following particular features:
- the fluid connection device consists of a fitment provided with an attachment body comprising a flange for attachment to an opening of a fluid containing device having an interior volume;
- the attachment of the flange allows entirely covering and sealing the opening, so that the interior volume is a closed volume.
- the interior volume serves for storage and/or circulation of the product.
- the filter device is disposed within the fitment in the inserted position and can be part of an inlet or outlet for fluid communication with the interior volume.
- the segment of conduit extends longitudinally from the free end of a the tubular male member (that is protruding from the attachment body, away from the interior volume,
- the segment of conduit extends longitudinally from the male member free end and said mouth where the lumen is in fluid communication with and/or opens into the interior volume.
- the fluid containing device may be a pouch/bag, for instance a single use 2D or 3D pouch, made sterile for instance thanks to a sterilizing operation.
- the flange of the attachment body is flat, possibly forming at least one planar inner face, and may be a plate.
- the flange of the attachment body can be an elongated flange of a boat port device.
- the outer face and the inner face of the attachment body are typically arranged in parallel.
- the tubular male member is arranged perpendicular to the plate.
- the attachment boy and the segment of conduit belong to a single piece of plastic, preferably defining at least two channels, one of the channel forming the lumen of the segment of conduit.
- the flange has two opposite flange ends, with the at least one channel being interposed between the two body ends.

The filtering assembly can be provided with one or more filter devices, each mounted in a tubular male member included in a fitment formed as an interface equipping a pouch, a bioreactor or similar containing device. In embodiments, the filtering assembly is a filtering assembly for a plastic bag having a flexible wall made of one or more flexible plastic sheets to delimit a closed and sterile chamber forming the interior volume. In some options, the filtering assembly may be provided with one or more of the following:
- a bag opening extending through the flexible wall and forming said opening (with the filter device arranged to filter liquid escaping from or entering the interior volume via the bag opening);
- the fitment provided with the attachment body in contact with and secured to said wall at the opening, so that the fitment or port fitment is closing/sealing the opening.

In preferred options, the plugging part is a mounting head of the filtering device in continuous annular contact with the segment of conduit. Additionally, when the segment of conduit can be integrally formed with the attachment body that comprises:
- the flange for attachment to one or two of the one or more plastic sheets of the plastic bag, the attachment body having a base at the flange with the base elongated along a given direction;
- an outer face, and
- an inner face arranged opposite the outer face, typically with the mouth provided in the base and opening to the interior volume through the inner face, so that the base is crossed by the lumen that extends perpendicular to the given direction.

The tubular male member can be made of plastic and/or protrude, along the longitudinal axis, from the outer face. The tubular male member can be entirely provided externally with respect to interior volume defined by the bag or similar fluid containing device. The structure including the male member can optionally have at least two male members for hose attachment, for instance with these male members arranged parallel and perpendicular to the attachment body.

A circumference/periphery of the attachment body can form a continuous fixation area entirely surrounding the opening. The attachment body may optionally include a thermoplastic layer for welding onto a matching layer of plastic film.
The inner face of the attachment body may be planar, possibly extending between two opposite ends of the attachment body. In variants, the inner face may be provided with at least one hollow, possibly with the mounting head adapted to axially contact the inner face by also being housed in a recess, groove or hollow of the attachment body. Two axial contacts of the mounting head, on different subregions of the inner face may be provided in some variants.

In some embodiments of the filtering assembly, one or more of the following features are provided:
- the filter device consists of the trap portion, the mounting head and a
- the mounting head has a fitting part that provides an annular sealing contact against a segment end of the segment of conduit.
- the mounting head, partly extending outside the lumen and thus having a mounting head outer face facing away from the filter insertion end (opposite from the inserting portion), is permeable to liquids and solids to be retained by the trap portion (filtering trap).
- the mounting head is provided with a maximal outer diameter or width of the filter device at an external part adjacent to the fitting part, the maximal outer diameter or width being greater than any internal diameter of the tubular male member, and preferably greater than a maximal diameter of the lumen.

The filtering assembly may comprise the plastic bag that is preferably a single-use bag or can comprise a wall portion, delimiting the opening and including at least one film, of the plastic bag. In embodiments, each filter device is mounted inside the lumen before an assembling of the plastic bag.
Following a first option, the filtering assembly has a port fitment or fitment provided with an outlet and:
- the attachment body and the male member are part of the outlet in the fitment carried by the plastic bag (or similar bag unit),
- and the mounting head/plugging part of the filtering device axially presses onto the inner face of the attachment body to maintain the inserted position of the trap portion (extending at a predetermined insertion level thanks to such axial abutment: such abutment co-act with friction obtained at a sealing annular contact to lock the insertion position).

In some conceptions compatible with the first option or variants where the filter device is embedded in a fluid outlet, a relief or groove may be present in interior face of the lumen, typically in the attachment body, to have a snap fitting. The fixation of the filter device may be permanent, with possibility to unlock the inserted position.
More generally, an axial contact of the mounting head may optionally be obtained on an inner shoulder, groove or on a protrusion, facing toward the interior volume and forming a subregion of the attachment body interior face extending around a male member central axis.

Following a second option, the filtering assembly has a port fitment or fitment provided with an inlet and:
- the attachment body and the male member are part of the inlet in the port fitment carried by the plastic bag (or similar bag unit),
- and the mounting head/plugging part of the filtering device axially presses onto an annular end of the tubular male member to maintain the inserted position of the trap portion (extending at a predetermined insertion level thanks to such axial abutment: such abutment co-act with friction obtained at a sealing annular contact to lock the insertion position).

The attachment body and at least one sheet of plastic film material are in an overlapping configuration. According to an option, the attachment body is sandwiched between two margin portions distributed in a first sheet and in a second sheet of the one or more flexible plastic sheets, with the attachment body having two opposite welding surfaces welded to the wall. In another option, the attachment body is a plastic plate selectively adhering to only one of the flexible plastic sheets, with the attachment body closing the opening (formed in/by this flexible plastic sheet) and having an annular attachment surface, preferably a welding surface welded to the wall.
When the attachment body is made of a plate; it is provided with an axial aperture to define an extension or end of the lumen. The plate can extend inside the interior volume of the wall or, alternatively, cant extend outside the interior volume of the wall, by adhering with an outer surface provided in fixing area of the flexible plastic sheet. This sheet carrying the connection interface may be optionally combined with another sheet of same size and shape to form the interior volume, either with use of gusset sheet(s) to provide a 3D bag, or without any gusset to provide a 2D bag.

According to a third option, the fluid interface connection may constitute or be a part of a tube to tube connection system. In such case, the fluid connection device can be enlarged in an end section away from the tubular male member. The tubular male member may be included in a tube-to-tube connector (possibly may as a monolithic structure, for instance single plastic or metal piece) or in a clamp system/assembly, for instance of the Tri-Clamp^{®} type. In embodiments in accordance with this third option, the fluid connection device may be one amongst a tube-to-tube connector and a single piece coupler of a tube-to-tube connection system. Additionally or alternatively, the lumen extends rectilinearly along the longitudinal axis and opens into a chamber for fluid circulation. Optionally, the mouth can be provided at a section transition region that is narrowed, possibly progressively narrowed, toward the segment of conduit.
When the fluid connection device belongs to a clamp system/assembly, it is understood that the plugging part is typically inserted in the lumen via an opening (single opening) formed at the free end of the tubular male member.

The fluid connection device can preferably be made of a single piece forming a coupler, so that it comprises at least two nozzles, preferably barb nozzles, facing in opposite or divergent directions.

Optionally, the tube-to-tube connector comprises an intermediate section between said nozzles and provided with a fluid passage allowing fluid communication between each lumen formed in a corresponding nozzle of the at least two nozzles.

According to an aspect of the disclosure, a filter device is provided that has a structure combining a filtration section and a fixation area, respectively distributed in a trap portion and in a plugging part. The filter device is intended to be mounted/inserted directly in a barb fitting. Following this specific aspect, embodiments of the disclosure provide a filter device that is provided with an insertion body having a insertion end and an outer end provided at the opposite from the insertion end, so that the filter device is insertable (as an unit filter) inside a barb fitting or similar tubular male member of a hose connection.

The filter device, which is adapted/intended to form an insert in a hollow fluid connection interface for a liquid biopharmaceutical product (containing complex products, living cells, drugs, ophthalmic care solution or the like), typically by insertion through an open free end of a tubular male member forming an inlet or by insertion from inside through a channel a tubular outlet, comprises:
- a plugging part that is permeable and carrying or including the outer end that is enlarged to provide at least one transition of section under the outer end, the outer end preferably forming a shoulder;
- the insertion body, which extends longitudinally from the at least one transition of section;
- the trap portion that forms the filtration section, for instance thanks to at least one amongst a membrane, a mesh and a packing of granular material, and
- an annular sealing element provided between the outer end and the trap portion, so that the annular sealing element is included in the plugging part, the insertion end being preferably a filter insertion end;
wherein the filter device is configured to selectively retain visible solid particles which have a size superior or equal to 100 micrometres, by trapping these visible solid particles in the trap portion that is elongated, along a longitudinal axis to be preferably longer than 3 or 6 mm.

The plugging part, typically forming a mounting head, may be:
- permeable to liquids and solids to be retained by the filtering trap;
- provided with a maximal outer diameter of the filter device, which is greater than an maximal diameter of the lumen.
The mounting head can be provided with an annular groove or recess to house an annular sealing element inside the lumen and adjacent a shoulder of the mounting head.

Preferably, the mounting head has a rear end flange part, forming a radially protruding lip. The rear end flange part has a maximal thickness inferior to 6 mm, preferably inferior to 4 mm. Additionally, the axial extension/radial size of the rear end flange part, in an annular margin portion around the opening area (open area to form the fluid inlet of the filter device), may be also inferior to 4 mm. More generally, a lip can be provided in the mounting head with the radial size of the lip and the thickness of the lip being inferior to a threshold that may be 2, 4 or 6 mm, while a diameter of the central opening of the mounting head can be at least twice superior to the threshold (for instance with the opening diameter being equal or close to the inner diameter of the hose intended to be attached to the male tubular member/barb fitting).

The central opening of the mounting head may have a diameter comprised between 3 or 3.2 mm and about 25 mm. The radial extension formed by the radial size of the lip may be inferior or equal to 2 mm.

The plugging part has a continuously rounded circumference at least at a sealing surface formed by the annular sealing element. In some options, the plugging part can have an outer skirt to delimit a hollow, in which a part of the trap portion, typically formed of filtering material, can be inserted. The trap portion is configured to purify the flowable biopharmaceutical product by separating and keeping the visible solid particles downstream the plugging part, between the plugging part and the filter insertion end provided opposite from the plugging part (forming the insertion end of the insertion body).
In conditions of use, the trap portion can extend inside a lumen of a tubular male member, typically in a barb fitting, for flexible hose attachment in a fluidic connection. The plugging part is a mounting head that can abut onto a surround of a lumen access, whereby the insertion body can be axially locked or maintained in inserted position by the plugging part.

One or more of the following filter device features can be provided, whatever the specific integration to filter the visible particles:
- The filter device is a single-use filter, not re-usable with another liquid.
- the filter device has a mounting head (formed as a plugging part) comprising a fitting part provided with a resilient material or with a resiliently deformable lip or bead of annular shape.
- the fitting part of the mounting heat (less wide than the outer end) can provide an annular sealing contact against a segment end of the segment of conduit forming the lumen,
- the mounting head is permeable to liquids and solids to be retained by the trap portion (filtering trap);
- the mounting head is provided with a maximal outer diameter or width of the filter device at an external part adjacent to the fitting part (is it understood that the maximal outer diameter or width can be greater than any internal diameter of the tubular male member, and preferably greater than a maximal diameter of the lumen receiving the insertion body).

In some embodiments, the filter device can be any insertable device fitting in any suitable male member of a fluidic connection member, thanks to annular fixation area provided in the plugging part provided as a permeable end, permeable to the visible particles that are then retained inside a volume (porous volume or central hollow area for instance) of the trap portion.
A filtration level of the trap portion may be configured to be higher than 100 µm. More generally, when a filtration membrane or a porous filtering mass/body (possibly including granular material) is present, no ultrafiltration and no nanofiltration is provided: only microfiltration (MF) is involved, with provision that no solid particle, product or living cell having a size inferior or equal to 50 µm is retained by the trap portion. In other words, the filter device prevents any particles having a size inferior or equal to 50µm to be retained.

The plugging part is or defines a filter inlet. In embodiments, the plugging part may be a hollow tubular end of the filter device. While the plugging part may restrict a passageway section and/or provide a prefilter to prevent some millimetric particles from entering inside the trap portion, it is understood that the plugging part is permeable to liquid and all visible sub-millimeter solid particles, thus not creating any significant loss in the flow rate (no pressure loss) at a filter device inlet, unlike ultrafiltration membrane or similar fine filter.
Moreover the trap portion can be longer than 3 or 6 mm, possibly much longer than 11 mm with a maximal outer diameter or width less than 6 or 8 mm. The filter device thus can be inserted in any lumen whose diameter is less than 20 mm, typically inferior or equal to 15 mm.

It is understood that the filter device can be surrounded by a barb fitting and by a flexible hose deformed and mounted on the barb fitting. Exemplary sizes of flexibles hoses (with values for inner diameter - outer diameter) that are surrounding the trap portion and the barb fitting forming the lumen are given below. Here no flexible hose inner diameter (nominal diameter) exceeds one inch (25.4 mm). Of course, other options for the flexible hose can be chosen, provided the filter device can be inserted for suitably filtering the liquid biopharmaceutical product.

**Table 1**

| Outer size of hose/tube | Inner size |
|---|---|
| Tube 1 with outer diameter of 34.9 mm (1-3/8") | 25.4 mm (1") |
| Tube 2 with outer diameter of 28.6 mm (1-1/8") | 19.1 mm (7/8") |
| Tube 3 with outer diameter of 19.1 mm (7/8") | 12.7 mm (1/2") |
| Tube 4 with outer diameter of 15.9 mm (5/8") | 9.6 mm (3/8") |
| Tube 5 with outer diameter of 12.7 mm (1/2") | 6.4 mm (1/4") |
| Tube 6 with outer diameter of 9 mm | 3.2 mm ( 1/8") |

According to some embodiments, the filter device (which can be compact) can include one or more of the following options/features,
- at least one filter outlet is provided on an outer circumference of the insertion body.
- the trap portion is delimited by a longitudinal face formed by a filtration membrane or filtration media.
- thickness of the filtration membrane or filtration media is less than 1,5 mm.
- the trap portion has a maximum diameter lower than 14 or 19 mm.
- the trap portion is longer than wide with a length of the trap portion at least twice superior to said maximum diameter.
- the plugging part is at least 3 mm wider than the trap portion, while preferably having a maximum outer diameter or width inferior to 30 mm.
- the plugging part includes a fluid filter inlet (forming an inlet of the filtering assembly), the fluid inlet communicating with a central hollow or with a porous structure of the trap portion.
- the mounting head/plugging part can be at least partly porous or provided with a hollow, with the porous or hollow part of the plugging part being in fluid communication with the inner volume defined by/resulting from the porous structure.
- the fluid filter inlet is an axial inlet, while a side wall of the trap portion is permeable and, optionally, configured to allow centrifugal filtration inside the lumen.
- the filter device has the insertion filter end and the plugging part constructed together as a monolithic part or included in a pre-assembled unit assembled before being mounted in the lumen.
- the filter device is constructed with the trap portion of outer size at least 2 mm narrower than the sealing element.
- the trap portion is entirely included in the insertion body/part.
- the filter insertion end is rigid and/or can be rigidly fixed to the fitting part.
- the filter device consists of the insertion body, the sealing element and the outer end (also called rear end), with the outer end being annular to axially open outside the lumen (and form the filter inlet).
- the plugging part has a central hollow and/or has a symmetrical shape around a central axis, with the liquid biopharmaceutical product flowing axially through the central hollow of the plugging part.
- the plugging part opens axially to the trap portion, at an annular junction provided to joint the plugging part to the trap portion.

In some embodiments, the trap portion is narrowing with increased distance from the plugging part. Additionally or alternatively, the plugging part may have a symmetry around a central axis.
When the trap portion is narrowing or has a tapering profile, a longitudinal filtering member may be provided. The filtering member, for instance a filtering part, media/layer or membrane, can either be included in a rigid structure of the trap portion, or be affixed to the rigid structure to cover a trap portion opening forming a filter outlet. For instance, the trap portion comprises at least one filtering membrane or media extending longitudinally, preferably with two elongated sides of the membrane or media. In some options, the two elongated sides are fixed to two triangular flanges or sides of the rigid trap portion. More generally, a side opening of the trap portion may be covered by a filtering layer that allows selective retention of the visible particles (with proper filtration level to let the lower size particles flowing through the filter device).
The two elongated sides may be parallel longitudinal sides extending between a first membrane/media end adjacent to the plugging part and a second membrane/media end provided in or adjacent to the filter insertion end.

In options with or without a membrane or similar media layer, the trap portion can include a cylindrical or tubular delimitation, provided with a first mesh and extending around a central axis perpendicular to a radial portion of the plugging part, the radial portion being provided with a second mesh. The first mesh allows finer filtration than the second mesh. An interior volume, upstream the filtration layer formed by or including the first mesh, is delimited axially between the radial portion and the filter insertion end, while being also delimited by a tubular wall having the second mesh.
More generally, the trap portion can be made of a component provided with a tubular mesh or a 3D porous structure that is rigid and performing retention of the visible solid particles inside a filter device interior volume.

In other options, in addition or as an alternative in conception of the trap portion, a granular material can be housed inside the trap portion, in the interior volume thereof delimited by the trap portion tubular delimitation. In some options, the granular material is filled between the plugging part forming a first plug and the filter insertion end constructed as a second plug, preferably identical to the first plug. Alternatively, the granular material is filled between the plugging part forming a first plug and a partitioning wall of the male connecting member, possibly constructed as a single piece with the tubular wall of the male connecting member. The partitioning wall can be less permeable than the plugging part, i.e. provided with a level of filtration that is finer/fine as compared to level of filtration allowed by the plugging part when provided with a grid or mesh.
More generally, the plugging part may be- co-acting with a transverse wall mounted inside the lumen to delimit a cavity receiving the filtering material (bulk material/ granular material preferably).

Whatever the precise structure chosen for the trap portion extending axially from the plugging part, the trap portion can be (in conditions of use) hidden, typically entirely hidden, inside a fluid connection interface, with the plugging part fitted inside lumen of the male member/barb fitting.

The trap portion can have a fillable cavity that can be (removably or irremovably) closed by a plug forming the plugging part. The plugging part, having a fitting part that provides the annular sealing contact against a segment end of the segment of conduit, is adapted to be partly inserted inside tubular male member (formed in a barb fitting). The plugging part can close the cavity for the filling of granular material, the granular material forming all or part of the trap portion.
In some embodiments, the granular material may comprise or consist of beads (typically of a same size/diameter, or alternatively of at least two distinct types). It is understood that the trap portion includes a housing, to house the granular material without any loss.
More generally, the filter device can be deprived from any intermediate support, distinct from the outer wall of the filter device, to link the filtering material or media to a housing of the trap portion.

In some options and variants, the filter device can be provided with the following features:
- the trap portion comprises channels formed by hollow guides having fibres.
- the channels, preferably arranged parallel along the longitudinal axis, are configured to allow for tangential flow filtration.
- the hollow guides may have each a thickness (in fibrous material) inferior to the internal diameter of the channels, such thickness being between 20 and 55 µm for instance.
- the hollow guides have first hollow guides provided internally in the trap portion and a cylindrical arrangement of second hollow guides that surround the first hollow guides and define an outer side surface of the trap portion.
- each of the hollow guides is elongated, possibly at least three times longer than the plugging part.
- each of the hollow guides have a first end housed inside the plugging part/mounting head (with the hollow guide first ends surrounded by an outer skirt of the mounting head).
- the trap portion comprises a porous rigid monolithic structure with inner pores distributed along and around the longitudinal axis in the trap portion.
- the rigid monolithic structure also has outer pores, in fluid communication with the inner pores, which are outer pores (opening in the lumen when the filter device in mounted in a connection interface for liquid circulation).

The filter device can be used/positioned at:
- interface of a container (bag for instance or bioreactor) and a tube;
- or in a tube line.
The trap portion is part of an insertion portion/body of the filter device, with the insertion body being entirely thinner than the mounting head forming the plugging part, which facilitates insertion/installation in a hollow of a tubular male member or similar connection forming a thin lumen. Besides, The trap portion may be non-deformable and/or constructed to be longitudinally/axially incompressible.

According to another aspect, a use of the filter device as above presented is provided, with the filter device included in a circuit or flowing path for filtering a liquid biopharmaceutical product, so that one or more final biopharmaceutical drug components, having molecules, biomedical compounds or cells (living cells) of characteristic size or diameter inferior to 50 micrometres, are included in the flowable biopharmaceutical product that is circulated through the filter device in the circuit or flowing path to be filtered (without any loss of said molecules, biomedical compounds or cells).
Typically, the filtered product can reach then a dose forming apparatus, without any subsequent filtering downstream the filter device, the dose forming apparatus being configured to deliver a dose made of the filtered product.

In embodiments, use is provided for the filtering assembly including the filter device (thus with the filtering assembly provided in a circuit or flowing path), wherein one or more final biopharmaceutical drug components (having molecules, biomedical compounds or cells of characteristic size or diameter inferior to 50 micrometres) are included in the flowable biopharmaceutical product that is circulated through the filter device in the circuit or flowing path to be filtered (without any loss of the special valuable molecules, biomedical compounds or cells).

In such application, the filter device is advantageously forming a part, typically compact/not exceeding 30 or 40 mm, easy to be embedded and providing a filtration that is efficient in terms of flow rate (remaining as high as wanted) and prevention of risk a final product is disposed due to pollution by visible particles (typically not forming reactive molecules) that can appear at a late stage in the bio process due to conditioning issues.

According to another aspect, embodiments of the disclosure provide a method for assembling a bag unit, chosen amongst a single-use bag or device (possibly a parenteral product bag, bag or transfer set device for gene therapy product, autologous cell therapy, heterologous cell therapy) and a bioreactor (preferably a single use bioreactor), with integration of a filter device through a lumen in a male tubular member, the bag unit including sheets of plastic material to form a wall delimiting a sterile interior volume, the method comprising:
- providing a fitment/port fitment forming a hollow fluid connection interface, through an of the wall, the fitment including a fluidic connection member provided with the male tubular member;
- inserting a filter device inside the lumen, in an inserted position, with the filter device having a plugging part, a trap portion configured to selectively retain visible solid particles which have a size superior or equal to 100 micrometres downstream the plugging part, between the plugging part and a filter insertion end provided opposite from the plugging part in the filter device; and
- maintaining the inserted position with the trap portion disposed adjacent to the plugging part, inside the lumen, by an axially locking action performed by the plugging part against a segment of conduit of the male tubular member where the lumen is delimited, in order to obtain the flowable biopharmaceutical product filtering assembly as above presented.

The bag unit can have a parenteral nutrition bag or a parenteral medication bag. Besides, the filter device can be a preassembled part, which facilitates integration of the filter device by only pushing the plugging part, without contacting the trap portion in the mounting step.
The parenteral product bag of this unit may consist of a parenteral nutrition bag having a bag outlet nozzle or a bag inlet nozzle included in the filtering assembly to delimit the segment of conduit.

Advantageously, the filter device allows delivering a final product by an outlet forming part of the filtering assembly, without any additional filtration step of the liquid biopharmaceutical product retrieved downstream the filtering assembly (according to the direction of circulation with the liquid entering the plugging part first, before flowing out of the trap portion).
The filter device can be used in any process involving water or an alternative solvent suitable for a parenteral injection.

### Brief Description of Drawings

Other features, details and advantages will be shown in the following detailed description and on the figures, on which:
Fig. 1 illustrates, with a longitudinal section view, a fluid filtering assembly with integration of filter(s) according to a first embodiment, here with a port fitment forming a fluid connection interface providing section(s) of conduit where a filter is mounted to have a trap portion for visible particles extending inside a narrow lumen and surrounded by the fixed hose end.
Fig. 2 illustrates an exemplary 2D bag carrying a filtering assembly allowing retaining visible particles, possibly with a fluid connection device and filter devices such as shown in Fig. 1.
Fig. 3A schematically illustrates components of a filtering assembly according to a second embodiment, with integration of the filter device in a lumen formed at a narrow end of one of the fluid connection components that belong to a three part clamping system.
Fig. 3B shows in a longitudinal view an assembled configuration of an elongated filter device that can have a tubular mesh, allowing a centripetal filtration, here in an option where the lumen is constructed as in the case of Fig. 3A, the filter device having a mounting head that axially locks/maintains position of the filter device taken as a whole in the assembled configuration.
Fig. 4 is a perspective view of a filter device (first embodiment) for retaining/trapping visible particles, with an elongated structure extending between a filter insert end and a plugging part configured to be in axial contact and in an annular sealing radial contact with a segment of conduit, so that the trap portion can extend inside the lumen of the segment of conduit.
Fig. 5 is a perspective view illustrating an exemplary insertion of two filter devices, here at a pair of inlets of a coupler forming the fluid connection interface, with the coupler having suitable barb fittings to provide respective tube-to tube connections.
Fig. 6A is a perspective view of different parts of a filtering assembly provided with a filter device including a granular material, for instance beads, packed in a hollow section (cavity of the segment of conduit) to form the trap portion
Fig. 6B is a longitudinal section view showing an exemplary mounted configuration of a filter device, using the parts shown in Fig. 6A, with a radial mesh or permeable wall formed through the lumen to provide an abutment and retaining surface of the granular material of the trap portion.
Fig. 6C is a view similar to Fig. 3B, in a variant where packed granular material such as used in Figs 6A-6B is present inside the lumen and sandwiched between two plugs that can be similar or identical.
Fig. 7 shows a non-limiting embodiment with use of filter devices to form a filtering part/assembly coupled to film portions of a single-use 3D bag/bioreactor.
Fig. 8 is a perspective view of a fitment adapted to provide two bag ports, with insertion of two filter devices in each lumen but using a different insertion direction, so that one trap portion can be arranged in a draining line, inside the fitment, while another trap portion can be arranged in a filing line, inside the same fitment.
Fig. 9 illustrates another embodiment (fourth embodiment) of a filter device, with hollow fibers included in the trap portion.
Fig. 10 illustrates an embodiment of a fluid vessel or bag, comprising a fluid port system that has a flange carrying barb fittings and filtering functions obtained thanks to filter devices mounted, either in an inflow direction in a barb fitting, or in an outflow direction in a barb fitting.
Fig. 11 schematically illustrates implementation of two filter devices in an option where the fluid connection device has a flange to be carried by a plastic bag or bioreactor, with each filter device having a porous/permeable structure integrally formed with a body of the plugging part and allowing a longitudinal filtration.
Fig. 12 illustrates an exemplary kit to form the filtering assembly, using a filtering device and a fluid connection device that provides several barb fittings, one of which is receiving the filtering device to form a fluid inlet of a coupler.
Fig. 13A illustrates a filter device according to a fifth embodiment, with a porous body that is typically either of a rigid polymer, or of a mineral material such as ceramic or metal.
Fig. 13B illustrates a optional structure that can be provided for the porous body, at least in the trap portion, for the fifth embodiment.

### Description of Embodiments

In the following for the word "radially", the point of reference is the longitudinal axis of the segment of conduit with "outward" and its derivates meaning "away from the longitudinal axis" and "inward" and its derivates meaning "towards the longitudinal axis". The term "insertion end" is used to designated a front end firstly inserted/being the first filter device part to be inserted through a lumen, matching with illustrated embodiments.

Detailed description of several embodiments of the invention is provided below, accompanied with examples and with reference to the drawings.
In the various figures, the same references are used to designate identical or similar elements. Length is to be understood in a usual way for the skilled person, following the longitudinal axis A (at least in assembled state) as described below.

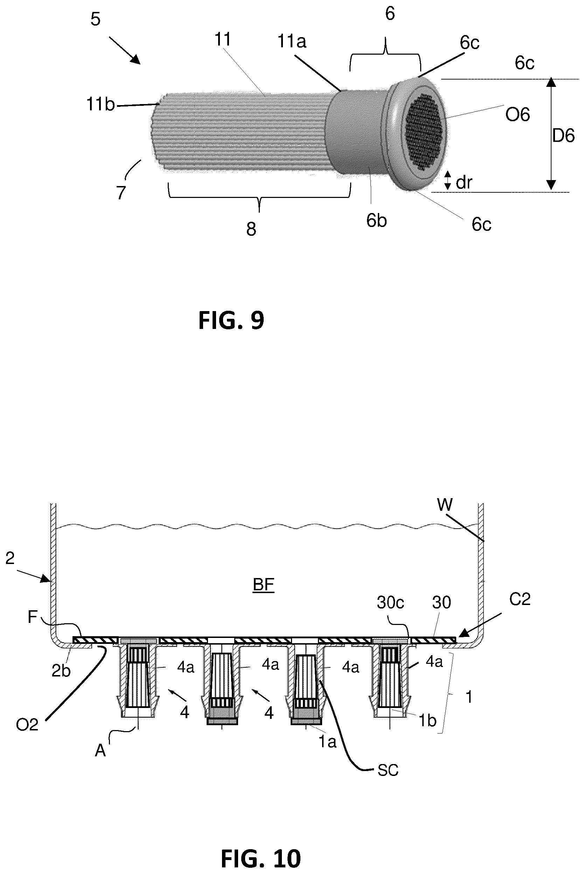

The disclosure is described for various options for filtration through a tubular male connector. Filtration can be performed in tube lines, bioprocessing systems or storage containers, where a flowable biopharmaceutical product BF (Fig. 10) is circulated through a line that extends from or to a fluid connection interface. In use, a filtering assembly 1, 101 can be formed by use of a filter device 5 that engages a segment of conduit SC of a coupler C2, C2', 20, 120 involved for fluid communication between a lumen of a flexible hose T and another fluid receiving region. The coupler C2, C2', 20, 120, forming the interface, thus comprises at least one fluidic connection member 4, provided with a hollow male member 4a at one side to have:
- a segment of conduit SC; and
- an outer annular face suitable for connection with an end of the flexible hose T.

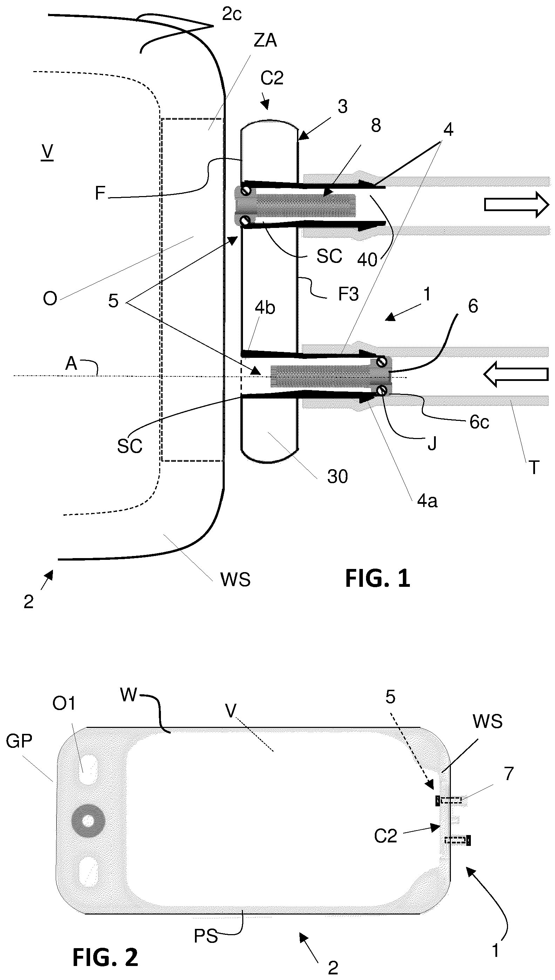

The filter device 5 can form a male insert, inserted inside the tubular male member 4a (thus in the lumen 40 of the segment of conduit SC; see for instance Figs 1, 3B and 10) to be coupled to a mating deformable end of a flexible hose T, with the deformable end being included in the resiliently deformable material of the hose T. The hose material can be a single layer material, which is biocompatible non-metal material, for instance a thermoplastic material or silicon.

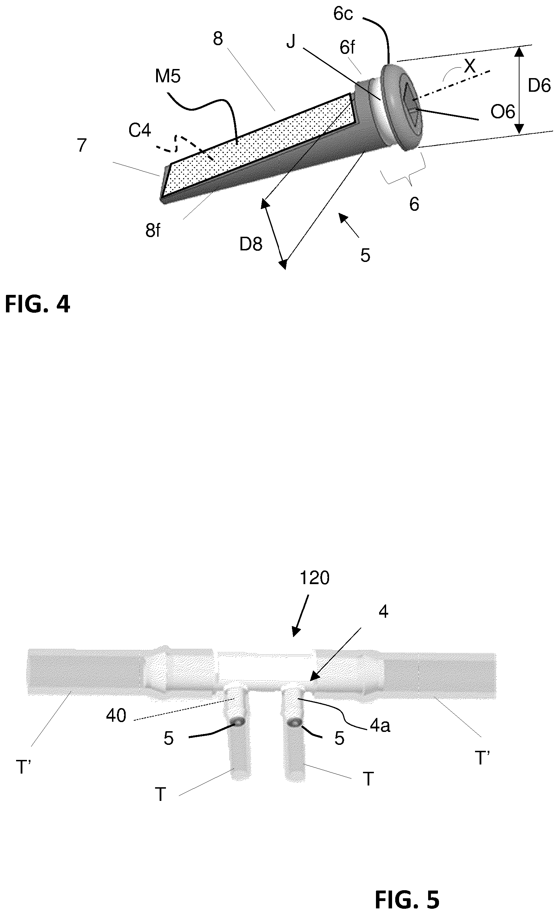

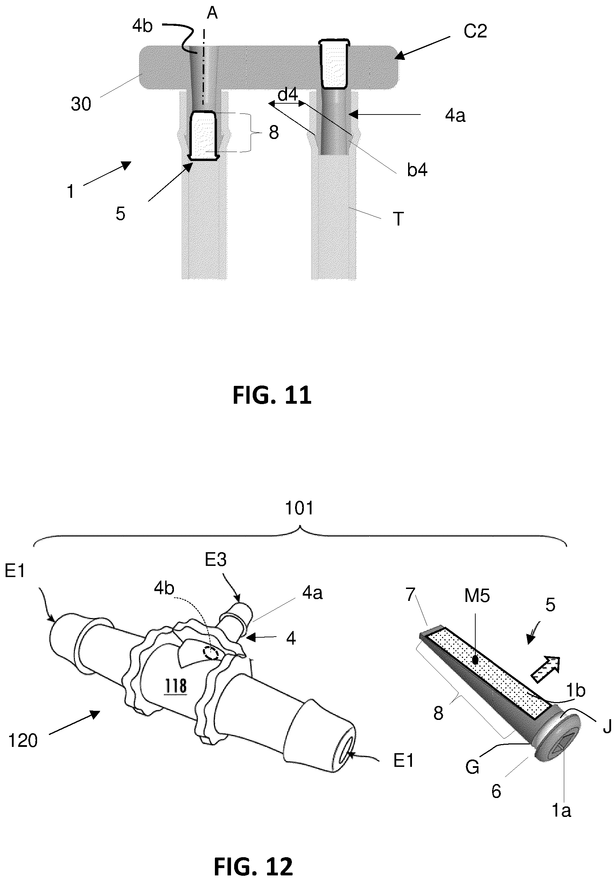

Each fluidic connection member 4 may be a barb fitting, so that a tubular male member 4a is provided for proper tight connection with a deformed end of the flexible hose T, as reflected for instance in Figs 1, 3B, 5, 6C, 7 and 11. Of course any similar tubular male member of a connection structure can be used to form a free end 4e and a lumen 40 that is forming a region for circulation of liquid surrounded by the flexible hose end TE.
For instance in Fig. 12, the tubular male member 4a can be provided with an opening of a coupler 120, here a T-coupler that defines three openings at the respective ends E1, E2, E3 of the coupler 120. The coupler 120 of Fig. 5 is similar but with four ends, with possibility to have a coupling with two additional hoses T', possibly of higher section and arranged downstream a pair of filter assemblies 1, with a filter device 5 respectively mounted in the two male members 4a that are radially protruding from the coupler body: here the coupler body extend between two opposite barb nozzles that are coupled to the hoses T'.
Such kind of coupler C2', 120 can have a fluid passage 118 so that each opening is in fluid communication with one another. The diameter may vary, for instance with at least one opening being comparatively smaller. The diameter provided at the ends E1, E2, E3 may be chosen to control the flow rate, the pressure or velocity of the biopharmaceutical product BF or any liquid passing in the coupler C2', 120 after circulating in a flexible hose/tubing.

More generally, the segment of conduit SC formed with a tubular member 4a may be part of a fluid connection interface having at least one male fitting such as a barb fitting for flexible hose attachment. In non-limiting examples of Figs 1-2 and 7, 10, it can be seen that the fluid connection interface may be directly constructed as a subpart of bag 2, with an attachment body 3 typically provided along a containing part of the bag 2.
Whatever the way the segment of conduit SC is constructed, forming a lumen 40 of constant diameter or with some transition(s)/inner shoulder(s), it typically defines a narrow lumen, i.e. not exceeding 0.5 or 1 inch (12.7 or 24.7 mm). The lumen 40 may have a circular section and/or extends straight/rectilinearly, by being delimited by a segment of conduit SC having a longitudinal axis A. A longitudinal direction can thus be defined at the segment of conduit SC, which also corresponds to an insertion direction: a plugging part/mounting head 6 of the filter device 5 can be guided by the segment of conduit SC when finalizing mounting of the filter device 5 inside the lumen 40.

The filter device 5 can be directly inserted inside the lumen 40 of the segment of conduit SC, linearly and preferably without any special rotational position. The plugging part may have a side contact part having a rotational symmetry. An insertion body of the filter device 5 can be provided with:
- a trap portion configured to be annularly surrounded by the tubular male member 4a when extending in the lumen 40 in installed position, possibly with a continuous spacing/annular gap; and
- a fixation region in annular contact with inner face of the tubular member at a position adjacent to the free end 4a of the tubular male member 4a.

The filter device 5 has a plugging part that is permeable and typically forming a mounting head 6. The plugging part can partly extend outside the lumen 40 in some options. The plugging part includes the fixation region, by having an annular sealing element J or similar contacting surface of annular shape, and an open end forming a fluid filter inlet 1a. The open end can be sized to define either an aperture O6 (as reflected in options of Fig. 4 or 9 for instance) surrounded by a frame forming a mounting head circumference, or a porous/permeable mounting head having a circumference (as in Fig. 11 for instance). In any case, the circumference of the mounting head 6 can have an outer diameter D6 (Figs 4, 9) or similar characterizing outer size lower than an outer diameter d4 of a bead/relief or barb b4 formed on the tubular male member 4a.
A lip or collar 6c can be provided on the mounting head 6 to have a rear end portion, at the rear of the annulare sealing element J, remaining outside the lumen 4, at least when the filter device 5 is inserted through a free end E1, E2, E3 of the tubular male member 4a to form a filtering assembly 1 of a coupler inlet or outlet. A coupler inlet is formed for instance by a male member 4a receiving a flexible hose T forming a supplying hose for a bag 2 carrying the coupler C2, as in the lower hose T shown in Fig. 1. In contrast, a coupler outlet can be formed by a male member 4a receiving a flexible hose T where liquid is circulated to escape from the interior volume V of the bag 2 as in the hose T shown at the top in Fig. 1 for instance.

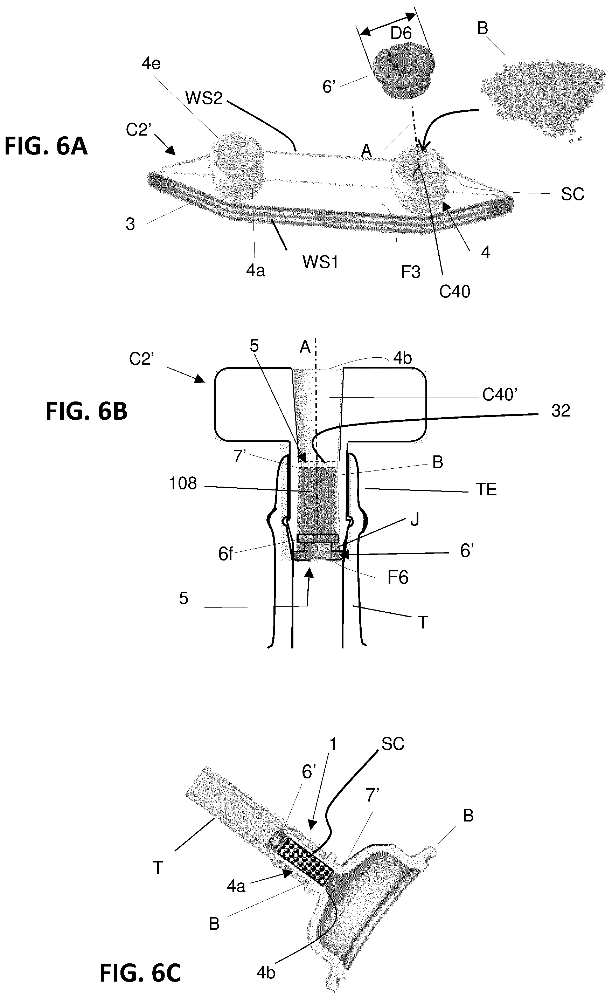

When installed in the lumen 40, the mounting head 6 of the filter device 5 thus can define the inlet 1a of the filtering assembly 1, 101, provided at (and possibly within) an opening mouth of the fluid connection interface provided in a suitable protruding male member. With such installation, the filter device 5 does not impair the attachment of the flexible hose T at the flexible end TE as reflected in Figs 1 and 6B, 6C in particular, even when the hose T surrounds the mounting head 6 constructed as the widest part of the filter device 5.
Typically, the filter device 5 can have a filter insertion end 7 narrower than the diameter D6, when constructed as an insertable preassembled unit distinct/produced separate from the fluid connection interface.

More generally, the filter device 5 can include a trap portion 8, 108 that is elongated, along the longitudinal axis A to be preferably longer than 3 or 6 mm, while being disposed adjacent to the plugging part and configured to purify the flowable biopharmaceutical product BF by separating and keeping visible solid particles downstream the plugging part, between the plugging part and the filter insertion end 7 provided opposite from the plugging part.
The trap portion 8, 108 is constructed and arranged to selectively retain visible solid particles which have a size superior or equal to 100 micrometres. As a result, the filtered liquid that is retrieved at a filter outlet 1b can be purified (without any solid matter/particles of such naked eye detectable size).

The trap portion 8, 108 extends inside the lumen 40, once the filter device 5 has been assembled and mounted with the plugging part engaging the segment of conduit SC, with an annular contact obtained at or adjacent to the end E1, E2, E3 of the male member 4a. The trap portion 8, 108 is axially locked or maintained in an inserted state/position by the plugging part.

The filtering assembly 1, 101 is interposed in a fluid path and includes the filter device 5 and the fluidic connection member 4, preferably without any intermediate sleeve or fixing part radially interposed between the segment of conduit SC and a filtering piece or media of the filter device 5, which is a filtering part included in the trap portion.
It is understood the fluid path may have any suitable liquid circulation structure away from the filter device 5 (the fluid path being an arrangement for conveying a fluid without exchanges with external environment). The fluid path typically comprises a flexible hose T (silicone or thermoplastic tube for instance) and a rigid segment of conduit SC provided with a conduit connecting fluid inlet or a conduit connecting fluid outlet.
Figs 1 and 2 reflect a fluid connection interface made of the coupler C2 that is suitable to form a port fitment (fitment that is a coupler 20 with the one or more male members 4a), once fixed to a wall W of bag 2 at a welding fixation area ZA. It is understood an opening O, O2 (as in Fig. 1 or in Fig. 10 for instance) in the wall W can be used to allow the biopharmaceutical product BF, in liquid form, to circulate toward the interior volume V or to be supplied outside this interior volume V, for instance to reach a dose forming apparatus / dosing means. The supplying hose, connected to a bag outlet having the filtering assembly 1, can allow circulation of the filtered product without any subsequent filtering downstream the filter device 5. In other words, it can be used for a parenteral bag or similar bag unit, in applications where one or more doses of the filtered product can be directly delivered (for therapeutical purpose).

The trap portion 8 and the plugging part forming the mounting head 6 may share a common single piece that:
- supports/holds or includes the sealing element J; and
- forms a support for or includes the filtering part of the filter device.
Referring to Figs 1, 3A-3B, 4, 6C, 12 and 13A, the plugging part may include a sealing element J and forms a mounting head 6, possibly constituting the only fixture portion for engagement with the segment of conduit, so that the trap portion 8. The common single piece defines a length of the filter device 5n which is an insertable device.

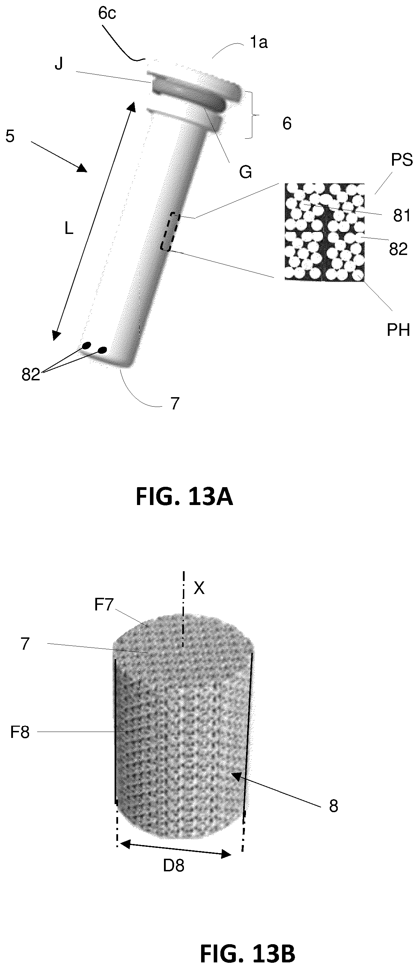

Preferably the diameter D6 cannot exceed a length L40 of the lumen 40 so that the filter device 5 is compact and elongated. In some options, the filter device 5 is provided with a trap portion length L (Fig. 13A) satisfying the following relationship: 0.4 < L/L40 < 1.2 , with the ratio L/L 40 being more preferably higher than 0.6 and inferior or equal to 1 or 1.1 The length L may be three times longer than the remaining length extension provided by the mounting head 6. Such length L may apply for any of the embodiments of the trap portion 8, 108.
The filtering assembly 1 can be compact, with a tubular outer surface of the trap portion 8, 108 defining a maximum diameter inferior to diameter D6 and lower than 20 or 25 mm. A transition or junction region is provided in the filter device 5 to rigidly connect the plugging part to the trap portion 8. At the filter insertion end 7, an axially permeable portion, possibly of rigid material (for instance, under form of a mesh extending transverse to the longitudinal axis A) , can be provided, where filtered biopharmaceutical product can escape from the trap portion 8, 108. Additionally or alternatively, the filter insertion end 7 is arranged to be spaced from inner wall/face of the segment of conduit SC or mouth 4b forming end of such segment so that filtered liquid escaping from the trap region (interior of the trap portion 8, 108), possibly radially by a radial/centrifuge filtration, can flow axially around the trap portion 8, 108 to join the mouth 4b.

An inlet and an outlet can be provided in the segment of conduit SC, at opposite ends thereof for instance, so that they are part of a same block, for instance with:
- the inlet access receiving the filter device 5 and surrounded by a flexible hose T at the barb fitting delimit the lumen 40 for insertion of the filter device 5;
- or vice versa, i.e. with the outlet access receiving the filter device and surrounded by a flexible hose T at the barb fitting, typically with the filter device by a rear or interior access of the lumen (opposite from free end of the barb end).
In preferred embodiments, as illustrated in Figs 1-2 for instance, a first male member can correspond to/be included in the conduit inlet (given fluid port) of a bag unit (bag 2) or similar fluid receiving device, while a second male member can correspond to/be included in a conduit outlet (other/distinct fluid port) of the bag/receiving device. All these ports allow attachment to a flexible hose T, which surround the filter device (by surrounding the mounting head 6 thereof and also the trap portion 8, 108.

Referring to Figs 1, 3A-3B, 5, 6A, 6B, 6C, 8, 10, 11 and 12, the segment of conduit SC can be constructed without any radial relief or aperture. The tubular male member 4a is typically arranged to be inserted inside directly in a lumen of a flexible hose T, with a tapering surface at or near the free end of the tubular male member 4a.
The member 4a and the annular part delimiting the mouth 4b can form the respective tube ends of this rigid elongated component, typically molded of plastic material (thermoplastic for instance), free of metal when provided in a bag or similar receiving part forming an interior volume for the product BF. The plastic of this elongated component can be compatible for contact with any biopharmaceutical fluid that can flow from a bioreactor or any path or circuit in a bioprocess. The mouth 4b in the segment of conduit SC is as wide or wider than opening provided at the free end of the tubular male member 4a, possibly with no variation or a variation of the cross-section that is limited/progressive to define a lumen 40 without any inner shoulder, through which the flowable biopharmaceutical product BF passes with suitable flow rate in operating conditions for fluid transfer.

### Options with the connection interface carried by a bag unit/fluid containing part

The wall of the segment of conduit SC is typically provided in a hollow part having a mounting structure/attachment body 3 suitable to be welded directly on at least one film of a container/bag 2. Such film is forming a flexible bag sheet 2c. The wall W of this bag 2, flexible, may delimit an interior volume V, possibly for storing the biopharmaceutical product BF in a liquid state. The wall W and a coupler 20, C2' forming the fluid connection interface CC are specially designed to contain a biopharmaceutical fluid. The coupler 20, C2' can form a quick connector for allowing a fluid connection with a flexible hose T. Of course a clamping part can be added to reinforce the connection/lock the connection. When having a clamp, tie or similar clamping part, the clamping area may be placed around (arranged coaxially with) the trap portion 8, 18.

Besides, the filter device 5 can have a rigid structure, without any folded or flexible part, typically compact to be housed inside the lumen 40 for 80%, 90% or more of the total length of the filter device 5. For instance, the filter device 5 can include a shell formed as a lattice structure, mesh structure and/or bearing structure carrying a fibrous filtering part, a membrane or filtering layer, preferably of deformable material. The filter device 5 is not axially compressible/deformable in preferred options, the filter device 5 having a rigid outer structure, unlike the sheet(s) 2c of the bag 2.

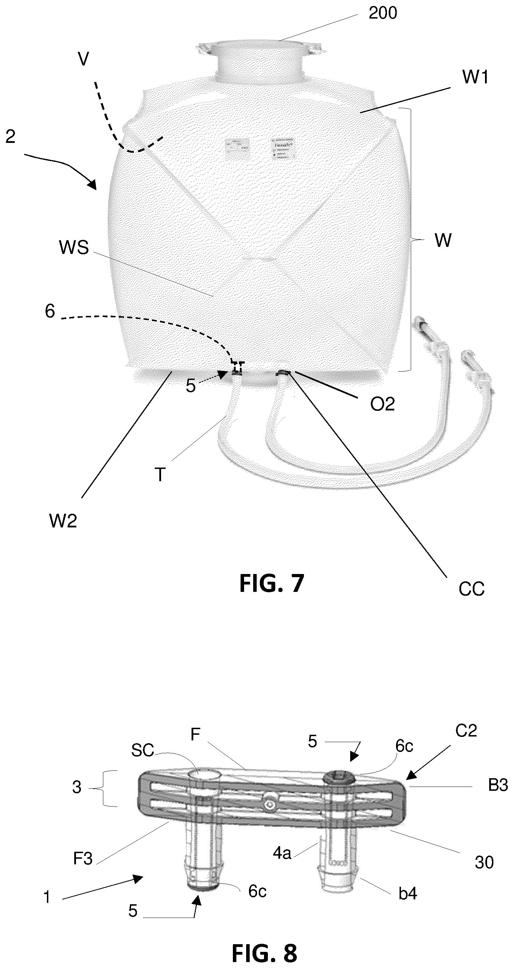

The bag 2 can have deployed/expanded configuration, starting from a substantially flat configuration. More generally, as in illustrated embodiments, the bag 2 has an interior volume V (Figs 1, 7) that can vary thanks to flexibility of the wall W. The wall of the segment of conduit SC extends tubular, preferably cylindrical, around the longitudinal axis A. This longitudinal axis A may be a central axis common for the male member 4a and the mouth 4b opening in the interior volume V The male member 4a can be a rigid hose barb connector and/or can form a nozzle, in order to constitute a bag inlet port or outlet port, allowing circulation of the product BF in a transfer mode. The filter device 5 can be embedded at such inlet port or outlet port, typically without any need to modify the way the flexible hose T is coupled to the male member 4a.
While a barb b4 or similar relief is shown in the drawings, more reliefs/barbs can used in some variants. Besides, the bag 2 that is collapsible, forming for instance a single use 2D or 3D pouch, can be replaced by any suitable bag unit having some non-deformable parts, for instance a base plate or top plate, or any fluid containing part provided with a port system or fitment part carrying at least one male member 4a coupled with the filter device 5.

In a first embodiment to form the bag 2, the flexible wall W is made of one or two flexible plastic sheets 2c and includes a U-shaped weld. As in the case of Fig. 1, a peripheral welding seam WS (possibly of rectangular shape) is obtained by welding two plastic sheets 2 together, so that the bag 2 can be formed with a wall W able to pass from a flat 2D configuration to an inflated configuration where the peripheral welding seam can remain substantially rectangular. The opening O is formed in the welding seam, typically at a rectilinear section of the peripheral seam PS (away from corner regions in Figs 1-2). This opening O allows forming the welding fixation area ZA for a port fitment. In other words, the opening O is able to receive at least one port of the flexible pouch/wall W forming the containing part of the bag 2.

Each filtering assembly 1 can have a filter device 5 inserted through the lumen 40 delimited by the tubular member 4a and a hollow of an attachment body 3 that carries the tubular male member 4a. The attachment body 3 can include or be a flange 30, elongated so that a pair of sheets 2c can sandwich the flange 30 in the welding fixation area ZA, with the flange 30 extending parallel to a side (optionally a rectilinear side) of the bag 2. When forming the fluid connection interface CC as a single piece having the flange 30 and one or more barb fittings or similar tubular male members 4a, a boat port device or similar coupler C2, C2' of elongated attachment body 3 can be obtained.
Referring to Figs 6A and 8, two exemplary boat port devices, each suitable to form the coupler C2, C2' are shown with a perspective view. The boat port device provides one, preferably at least two tubular male members 4a. Additionally, two welding surfaces WS1, WS2 are provided on two opposite sides of the attachment body 3, optionally with hollow recess(es) or grooves provided to separated two sub-areas for fixation with a corresponding plastic sheet 2c.

The segment of conduit SC can be a full conduit of a boat port interface or similar coupler, running from the inner face F to the free end 4e. The segment of conduit SC receiving the filter device 5 may be integrally formed with the attachment body 3 (Fig. 8) that comprises:
- a base B3 at the flange 30, preferably with the base elongated along a given direction;
- an outer face F3 (provided outside the bag 2); and
- an inner face F arranged opposite the outer face F3;
The mouth 4b of each segment of conduit SC is provided in the base B3 and can open to the interior volume V, through the inner face F. Since the tubular male member 4a protrudes, along the longitudinal axis A, from the outer face F3, the filter device 5 can be elongated and typically have a size higher than the tubular male member length, while being possibly shorter than length of the segment of conduit SC (which can define a maximum length of the coupler 20, C2, C2' as measured along the longitudinal axis A).

Now referring to Figs 7 and 10, the bag 2 can have a 3D pouch arrangement made by combining several flexible plastic sheets 2c. For instance, a sided K-weld design as in a 3D pouch commercialized with name Flexsafe^{®} bag can be used. More generally, welding bands/parts can be formed to have a 3D pouch, combining two main wall parts/films/sheets W1, W2 (for instance a top wall and a bottom wall) and two gussets parts, as in Fig. 7.
In a second embodiment to form the bag 2, which preferably has a 3D wall able to reach a substantially parallelepiped configuration in a filled state (with interior volume V filled with liquid), the fluid connection interface 4 can close a bag opening extending through the flexible wall W, for instance in one amongst a top wall W1 and a lower wall W2, forming an opening O2 typically spaced from any of the welding areas (spaced from any welding portion/welding seam WS).

As shown in Fig. 7, the attachment body 3 carrying the tubular member(s) 4a can be a plate or flange, with a plate face forming an annular junction where the connection interface CC is adhering to the plastic sheet/wall part of the bag 2, whereby the attachment body 3 formed as a plate covers the opening O2. Each trough-hole in the plate is in alignment with the tubular male member 4a, to define an elongated lumen 40 where the filter device 5 is properly inserted and fixed thanks to the plugging part. The trap portion 8, 108 can be either elongated but sufficiently short to extend only inside the tubular member 4a, or elongated to extend through both the attachment body 3 and a tubular member 4a rigidly and permanently secured to or integrally made with the attachment body.

When the filter device 5 is used to filter the product BF that has been stored/received in the volume V, the mounting head 6 or 6' of the filtering device can axially press onto the inner face F to lock the inserted position of the trap portion 8. An outlet port, equipped with the filter device 5, can be formed. The flow direction in the lumen 40 can thus be colinear with an insertion direction of the filter device 5 and the mounting head 6 defines/includes the filter inlet 1a at the permeable face/opening O6. The insertion end 7 is the proximal part (axially close/less distant) of the filter device relative to the free end 4e of the tubular male member 4a in such situation. The attachment body 3 and the fluidic connection member 4 (forming the male member 4a) belong to an inlet/inlet port in the fitment/coupler C2, C2' carried by the plastic bag 2 and closing the opening O or O2.

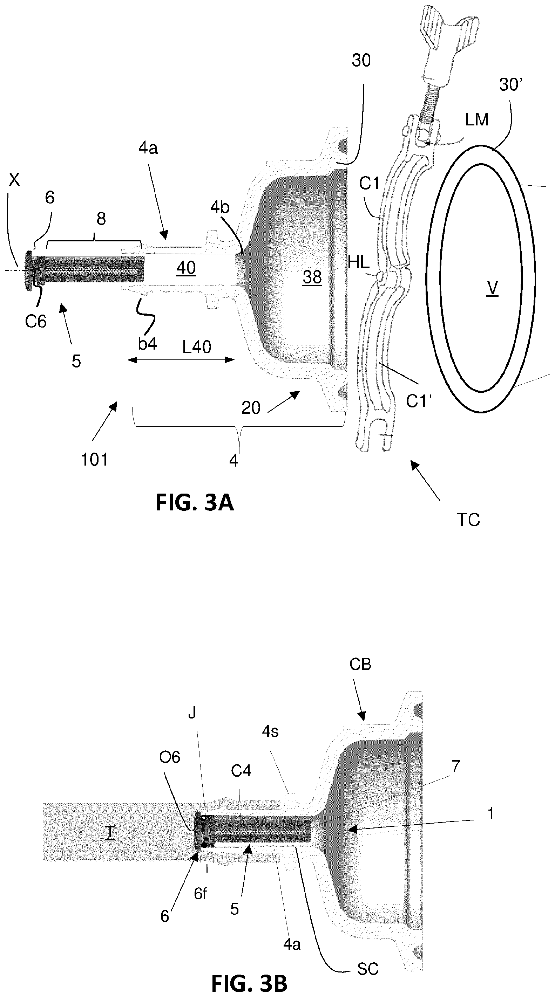

In another case, the attachment body 3 and the fluidic connection member 4 (forming the male member 4a) belong to an outlet/outlet port in the fitment C2, C2' carried by the plastic bag 2 and closing the opening O or O2. The flow direction in the lumen 40 of the outlet port can also be colinear with an insertion direction of the filter device 5 with the mounting head 6 including the filter inlet 1a at the permeable face/opening O6. It is understood that the insertion end 7 is the distal part (axially distant) of the filter device relative to the free end 4e of the tubular male member 4a, in such situation (as in the filtering assembly 1 shown on the top in Figs 1-2 or the one shown on the right in Fig. 8 / see also the assembly 10 of Figs 3A-3B).

In some constructions, the filter device 5 can have a relative short lip 6c or collar, as reflected in Fig. 8, so that a diameter D6 of the mounting head 6 is shorter than a shorter outer size of the attachment body 3. The mounting head 6 can optionally be provided as an integral extension extending the trap portion 8 or be replaced by a mounting head 6' covering the trap portion 108 as in Fig. 6. Independently from the size, shape and connection arrangement chosen for the mounting head 6, a filtering assembly 1 can be always mounted to have the trap portion extending outside the interior volume V of the bag 2, designed as a 2D or 3D flexible bag or as a partly rigid/reinforced bag. Fig. 1 shows that the trap portion 8 can be in overlapping condition relative to the welding seam WS but without extending inside the interior volume V. As a result, the filter device 5 has no significant impact on the flat initial configuration of a flexible bag 2 able to be deployed/inflated due to the filling.

The filtering assembly 1 can comprise a wall portion of the plastic bag 2, delimiting the opening O or O2. The flexible hose T can be also a part of the filtering assembly 1. Optionally, the flexible hose T can form a loop that is used to reinject a filtered product (filtered by the filter device 5 at a bag outlet) inside the interior volume V thanks to another port forming an inlet, equipped or not with an auxiliary filter device. A bag unit can be obtained with an integrated filtering function.
While the filtering assembly 1 can be provided at the opposite from a handle part GP of the bag 2 (adapted to form a top when the filtering assembly 1 is included in a final filling line, at the bottom of the suspended bag), as reflected in Fig. 1 as a non-limiting example, it is understood that the opening O or O2 may be placed in various manner in the wall W of the bag 2. One or two openings O1 may be provided in the handle part GP. A complementary port, possibly for a venting action, may also be provided close to the handle part. More generally, any bag/bag unit can be provided with a port for gas circulation (gas inlet or gas outlet) and/or venting of the interior volume.

### Exemplary mounting structure for integration of one or more filter devices

The segment of conduit SC can be made by a single wall component to delimit a single interior volume with the lumen 40 possibly at least twice narrower than a minimal transverse size of the interface defined in the mounting structure/attachment body 3. The plastic material of the segment of conduit SC can combine chemical resistance and sterilizability. Non-limiting examples are: polypropylene (PP), polyethylene (PE), polyvinyl chloride (PVC), and polycarbonate (PC). They can be typically used due to their compliance with industry regulations. Of course such choice of material can apply for any of the other embodiments, such as those describe hereinafter.

The segment of conduit SC can have a smooth inner surface of annular section, typically circular. An annular seal/sealing element J carried by the filter device 5 can be engaged inside the segment of conduit SC to be in annular sealing contact with the inner surface. Axial displacement, along same direction as the flow of the biopharmaceutical product BF in conditions of use is prevented by the lip or collar 6c provided on the mounting head 6. As a result, the mounting head 6 is plugged inside the segment of conduit with an external portion of the mounting head that remains axially engaged on a front end or alternatively on a rear end of the hollow male member 4a, outside the lumen.

It is understood the trap portion 8 can be part of an insertion body configured to extend inside a rigid tubular male member 4a (of an inlet or outlet of a coupler/fluid connection interface CC) so that, in a connected state the flexible hose T surrounds the tubular male member 4a and the trap portion 8: a filtering chamber can be selectively formed in the narrow lumen, without extending beyond the mouth 4b (lumen inner mouth) for instance.
The filter device 5 can made of biocompatible material, such as thermoplastics, ceramic, metal (stainless steel for instance), preferably with a rigid structure that only includes one material.

In some options, the lumen 40 has no partitioning wall or inner wall, which allows inserting a trap portion 8 without any obstructing part. In variants, for instance when defining a pair of cavities C40, C40' separated by a mesh 32 (as in some options reflected for example by Figs 6A, 6B, 6C), length of the trap portion cannot extend beyond the mesh 32 acting as a barrier for the filtering medium. The mesh 32 can here define a single partitioning wall for the lumen 40.
The filtering assembly 1, 101 can optionally combine the filter device 5, having the trap portion 8, 108, with the mesh 32 provided as prefilter or end filter, possibly having a hole size between 100 and 500 micrometres.

The filter device 5 can be preassembled to be inserted as an entire block/unit, in the lumen 40. Referring to Figs 6A-6B, it can be seen the filter device 5 can be not preassembled, typically with the mesh 32 being an integrated mesh of the connection interface (here with the mesh 32 included in the coupler C2').

Of course, a series of filter devices can be also inserted in some variants, either using an intermediate relief of the segment of conduit SC or the mesh 32 for abutment of a first inserted filter device mounted in a front/first position, while a second device can be also inserted in a rear/second position. In some options, the lumen can have compartments, with two of the compartments being filled by at least one of:
- a filtering media,
- a filter device/insert with mesh and/or membrane,
- a filter device with fibrous media or means for tangential filtration.
More generally, at least one filter device 5 can be formed and properly positioned, with a fixation allowed by a plugging part/mounting head 6: the filtering means of the filter device are axially blocked thanks to the plugging part (here a mounting head 6 with a lip 6c that remains outside the lumen 4) forming a rear end of this filter device 5. The wording "rear" or "at the rear" being here meant with reference to an insertion direction of the mounting head 6. At least when the filter device 5 is constructed as an insert, it is understood the filter insertion end 7 is the front end of the filter device 5.

In some embodiments, the filtration inside the lumen 40 by the trap portion 8, 108 is performed with use of mesh apertures, interstices, pore sizes adapted to prevent capturing of solid components having a size superior to 20, 40 or 50 micrometres. The solids retained in the trap portion can include filaments or parts of cellulose, textile fibres. Such solids consist of particles generally being of size greater than 200 micrometres, typically superior or equal to 400 micrometres for 95% of them.
Whatever precise integration of the filter device 5, it provides filtration of free visible particles (> 100 µm) to properly prevent contamination of final drug products or the like in the liquid biopharmaceutical product. In biotechnology processes, such filtration can occur before a controlling phase where particle contamination is detected, either by human eye or by an automated camera detection. No visible particle, i.e. visible *per se* due to its size will be present after such filtration by the filter device.

The filter device 5 can have a side wall, formed in a tubular component that is liquid permeable and provides the filtration range. The mounting head 6 or plugging part typically extends the tubular component, possibly with a conception in one piece to have the outer end (rear end) of the mounting head included in the piece forming the tubular component. The sealing element J may be the only other part, made of a different material, when integrating a mesh/mesh holes or a porous structure directly in the tubular component. The trap portion 8, 108 may be provided with an outer face F8, directly formed in a side wall of the trap portion. Diameter D8 of the outer face F8 may be of few millimetres, inferior to 20 mm for instance. An axial face F7 with mesh holes may be preferred at the filter insertion end 7 when:
- a cylindrical or tubular shape is provided for the side wall of the trap portion 8,
- or when the trap portion 8 is formed by a porous monolith.

### First embodiment of a filter device, with a mesh tube or similar filtration tube

In embodiments of Figs 1, 3A-3B, the number of pieces to form the filter device 5 is reduced, for instance with only two pieces assembled when the sealing element J is an annular ring of elastomer or similar resiliently deformable material. The filter device 5 can have a cylindrical insertion body with the sealing element J carried at a rear end of the insertion body, while the trap portion 8 delimits a central hollow C4. The filter insertion end 7 can be an axial closing end closing the central hollow, with or without axial openings provided in the filter insertion end 7. The optional lip 6c or similar radial protrusion of the plugging part rear end, provided around the axial opening O6, can ensure insertion of the filter device 5 is matching with the lumen 40, to prevent the filter device 5 extending beyond the mouth 4b or limiting the protruding extension of the trap portion 8 relative to the mouth area. The lip 6c can be annular with a contact surface perpendicular to a central axis X of the insertion body, so that in an engaged configuration of the filter device 5:
- the trap portion 8 is hidden inside the lumen 40,
- the lip 6c extends perpendicular to the longitudinal axis A of the segment of conduit SC that is insertable in a flexible hose T,
- the lip 6c and an annular rim formed at the free end 4e have mutually contacting surfaces extending parallel, while the sealing element J (or similar cylindrical/annular surface) is placed in radial contact (continuous annular contact) with an inner circumference area of the segment of conduit SC.

In Figs 1 and 3B, the fitting part 6f of the mounting head 6 includes the sealing element J and tightly separates the lumen region for circulation of filtered liquid product from an inside region of the hose T coupled to the tubular male member 4a. The trap portion 8, in stainless steel or rigid plastic material, can have a hole size between 50 or 100 micrometers and 500 micrometers in non-limiting embodiments.

The central hollow C4 can be an inner compartment, provided in the trap portion 8 provided with radial openings formed in the mesh structure delimiting the central hollow C4. More generally, the filter device 5 can allow radial filtration, which is centrifugal, inside the lumen 40, with also an axial filtration at the insertion filter end 7 when axial front openings are also provided. An access compartment C6 may be formed by the plugging/mounting head 6 (Fig. 3A), in alignment with the central hollow C4 so that the filter device 5 can be hollow substantially all along its length. It is understood that the size for each of the radial openings in the mesh structure is in the sub-millimetre range but typically much higher than 40 or 50 micrometres.

The plugging part is for instance at least 3 or 4 mm wider than the trap portion 8 or than the central hollow C4 of the trap portion 8, while having a maximum outer diameter D6 or width inferior to 30 mm, possibly superior to 3.5 or 4.5 mm. The plugging part preferably includes the fluid filter inlet 1a (forming an inlet of the filtering assembly 1, 101), which is possibly provided with a transverse mesh allowing axially separating the inner compartment defining the central hollow C4 and the access compartment C6 surrounded by the sealing element J and/or the lip 6c
While a filter device interior volume that is of substantially constant diameter is shown in Figs 1 and 3A-3B, at least for the central hollow C4, so that the trap portion 8 has a cylindrical geometry (with symmetry around axis X), the trap portion 8 can have a different geometry, for instance with a tapering or a transition in the cross-section. Besides, a monolithic construction can be preferred for the filter device structure including the filter insertion end 7 and the rear end (here with a lip 6c or similar axial retaining part) to prevent any accidental disconnection and simplify production steps.

In the cylindrical mesh forming the trap portion outer wall, the mesh holes may be distributed in several longitudinal rows, optionally with more than 10 or more than 16 holes per longitudinal row. Geometry of the mesh holes can follow one or more patterns amongst a circular section, a square, polygon (possibly with rectangular delimitation), ellipse, star or similar branched configuration.
Preferably, the density of the mesh holes around the central hollow C4 is sufficient to have the flow rate not unfavorably impacted (or with low decrease) by presence of the filter device 5, with the number of holes being, optionally, higher than 40 holes/cm² and typically lower than a threshold (which can be 250 or 350 holes/cm²). Whatever the precise number of mesh holes, mesh holes can be typically distributed to form a large number of longitudinal rows, possibly with more than 15 rows on the whole circumference of the trap portion 8. Besides, a quincunx distribution may be preferred to narrow the maximum interspaces between the holes and/or increase the section for passage of the filtered liquid product.

When provided with a base face (of circular section preferably) and a lateral face, the trap portion 8 can have a density of the mesh holes that is equal for the base face at the filter insertion end 7 and for the lateral face (spaced radially from the inner face of the segment of conduit).

### Second embodiment of a filter device, with at least one membrane or media

Referring to Figs 4 and 12, the trap portion 8 allowing the visible particles retaining/trapping effect is different from the first embodiment, with a media layer or membrane M5 integrated, while the plugging part may correspond to a certain extend to the above described plugging part with a sealing element J and an enlarged rear end. The fitting part 6f is in sealing contact with the segment of conduit SC as in the first embodiment at a filter device rear part (fitting part 6f), while the trap portion 8 can extend with an annular gap spacing this portion 8 from the segment of conduit SC. The plugging part can form a mounting head 6 with an axial fluid access at the opening O6, with the access compartment opening in the inner compartment (central hollow) delimited by the outer wall of the trap portion 8. The filter device 5 can form an insertable preassembled device.
The trap portion 8, elongated, can have a structure made in the rigid piece carrying the sealing element J. In the first embodiment and the second embodiment as well, an outer groove G can be provided, circumferentially, to receive the sealing element J typically formed as an O-ring. Such design can apply for any of the mounting heads 6, 6' illustrated/provided for maintaining inserted position of the filter device 5.

Regarding the filter device 5 of this second embodiment, the sealing element J is axially fixed to extend rearward relative to the membrane M5 or similar filtering media (with a fine mesh flexible structure or porous membrane). The membrane M5 is secured to the outer wall included in the piece, in order to cover an opening of the insertion body, which is here an elongated side opening. The elongated side opening is covered by the membrane M5 to form a filter outlet 1b directly equipped with the filtering media, here a membrane M5 that may have thickness of a film, possibly a sub-millimetre thickness or a thickness of about 1 mm. More generally, the insertion body of the filter device can gave one or more side openings each covered by a media/membrane M5, preferably with an elongated extension for the membrane M5.

Here, the rigid piece carrying the sealing element J has a tapering part tapering at the opposite from the filter inlet 1a, so that the filter insertion end 7 is narrower than a trap portion front section, of annular shape, provided adjacent to the fitting part 6f of the mounting head 6. The elongated structure of the insertion body, extending between the filter insertion end 7 (at the front) and the plugging part forming the mounting head 6 (at the rear) is configured to be:
- in axial contact and in an annular sealing radial contact with the segment of conduit, at the fitting part 6f,
- provided with two side portions 8f provided in opposite angular sectors of the trap portion outer wall, the two side portions extending longitudinally from the fitting part 6f to form two long sides of a frame region where the side opening is delimited.

In non-limiting option of Fig. 4, there is only one side opening provided covered by the membrane M5 that filters the liquid to separate visible particles of size higher than 100 micrometers. In variants or options for the second embodiment, more than one membrane M5 or similar media is provided. Possibly, the two sides 8f can delimit a pair of side openings covered each by a membrane so that a V-shaped filtering part is provided in trap portion.
A rectangular delimitation for the membrane M5 may be preferred, to cover a rectangular side opening delimited axially adjacent or close to the filter insertion end 7.

More generally, the filter device 5 can be designed to have a filtering part carried by the outer wall structure to cover a relatively wide side opening. Integration of the filter device in accordance with such second embodiment can be obtained for same range of couplers/connectors as those applying for the first embodiment. For instance, the bag 2 as in Figs 1, 7, 10 can be provided with one or more of the filter devices 5 that are readily/directly insertable in a matching tubular male member 4a. The same can apply when the tubular male member is a distal end of a connection device having several barb fittings or including two or more separable parts, such as in a tri-clamp^{®} system (see Figs 3A-3B).

When the trap portion 8 includes at least one filtering membrane M5 or media extending longitudinally, with two elongated sides 8f (parallel longitudinal sides) used to fix the membrane M5 or media, the filtering part (membrane M5) covering the side opening can be made/consist of polymer material or metal or ceramic with hole size typically between 100 µm and 500 µm, depending on the particles to be retained in the central hollow C4.
The tapering profile is configured to be a progressive tapering, without recess or enlargement typically, so that the fitting part 6f, cylindrical and/or provided with the suitable outer diameter D8, can be wider than the tapered portion but less wide than the rear end having the lip 6c with the maximum outer diameter D6 or similar radial size maximum.

Possibly, the membrane useful surface, at a covering portion of the surface where each side opening a surface is covered by the membrane can be provided to represent at least a third of the whole surface of outer side wall of the trap portion 8. More generally, a surface ratio between the membrane useful surface and the whole surface in the trap portion outer side wall can be higher than 1:4.
Of course, the membrane material or any filtering material used in the trap portion can be made of a solid layer only composed of insoluble and preferably chemically stable elements, not soluble in water/aqueous solvent.

### Third embodiment of a filter device, with packed material

All or part of the trap portion 108 may, in some options, include a granular material or similar bulk material, for instance beads B, adapted to define a porous mass, filling a hollow section (longitudinal cavity for instance) of the filter device 5, downstream a plugging part and upstream an end barrier element that can also be a plugging part.
The lumen 40 of the segment of conduit SC can provide proper location for housing the filter device 5 according to this embodiment (third embodiment), which means the plugging part may correspond to a certain extend to the above described plugging part, possibly also with a sealing element J. The fitting part 6f can be in sealing contact with the segment of conduit SC. For such reason, reference is made in particular to the differences with regard to the trap portion 108 and the way the beads or granular material is maintained in a hidden position, typically to be surrounded by an end TE of the flexible hose T coupled to the tubular male member 4a.

Referring to Figs 6A, 6B and 6C, the beads B or similar dispersed material that is a solid insoluble in water, with chemical stability: they may consist of an inert polymer or a mineral material, for instance ceramic or metal. The beads, preferably forming small spheres of diameter superior or equal to 50 microns (and typically lower than 900 microns), can block the visible particles when packed to form a compact mass of porous media (Fig. 6B). The trap portion 108 can be formed by a three-step phase:
- a) providing a bottom that delimits a suitable cavity, inside the lumen 40;
- b) filling the granular material in the cavity C40 (or C40') that has the bottom, which is either a permanent bottom formed by the mesh 32 or a removable bottom consisting of a plug 7' (as shown in Fig. 6C); and
- c) closing the cavity by the fitting part 6f of the mounting head 6'.
The plug 7' may have a structure similar or identical to the mounting head 6', possibly with lip 6c that engages an annular rim region delimiting the mouth 4b.

To limit the number of pieces/parts, a liquid permeable partitioning wall, here formed by the mesh 32, can form an obstructing and bottom part of one or two cavities C40, C40', directly inside the tubular male member 4a or -more generally - in the fluid connection interface/coupler C2'. While option illustrated in Figs 6A-6B is shown with the coupler C2' adapted to formed a boat port interface/device, it is understood that such configuration to define a cavity C40, C40' can be used in any of the others options, for example for a coupler/interface CC having a plate-like flange 30 to be attached to a bag wall (Fig. 7) or for a coupler C2' including the tubular male member 4a to form a hose coupling member distal from an annular base B serving in a sterile clamping connection (Fig. 6C).

In options to filter a liquid product circulating in the hose T before reaching the lumen 40, the plugging part/mounting head 6' is preferably provided with a fitting part 6f (as in Fig. 6B) that provides an annular sealing contact against an end of the segment of conduit SC, near the free end 4e of the male member 4a. The plugging part, constructed as a mounting head 6' initially separated from the trap portion 108, can be partially inserted inside the tubular male member 4a, away from the mouth 4b, after a filling of the granular material/beads B in the cavity C40 retained by the cavity bottom formed by the mesh 32.
As in the other embodiments, the mounting head 6' can be constructed as plugging part combining an axial abutment function, a sealing radial contact, and central access compartment for fluid communication with the initially empty hollow corresponding to the cavity C40 or C40'. But here, the trap portion 108 can be obtained without any specific outer side wall of tubular shape (remaining spaced from the section of conduit in the inserted position).

### Fourth embodiment of a filter device, with hollow fibres included in the trap portion

Referring to Figs 9-10, the filter device 5 can have a structure where the plugging part is substantially as simple as in the third embodiment, but with a specific fibrous structure of the trap portion 8 and a permanent connection of the filtering part forming the elongated trap portion 8. Typically, the fibrous structure has an end connection provided in an inner volume of the rigid piece, axially permeable, that forms the plugging part. In some conceptions, the plugging part has a plastic piece overmolded onto or welded around a hollow fibers/guides bundle.
Either a sealing element is carried by this rigid piece, or an outer lip/relief or bead is provided for an annular contact with the segment of conduit SC. A single plastic piece of the plugging part, forming the opening O6 and directly connected to the bundle (forming the trap portion 8), is provided, made of soft material (e.g. TPE, silicone). The mounting head 6 may be optionally designed as a single piece. In some variants, the mounting head 6 can be an assembly of at least two or three pieces, one of which can surround a rear end of the filtering part.

Here, the trap portion 8 comprises channels formed by elongated hollow fibrous guides 11 (having fibres or fibrous structure). The guides 11 may be made each of a hollow fibre/fibrous arrangement of strands or made of hollow fiber membranes. The hollow fibrous guides 11 can have a separating/filtration effect substantially like in a dialysis filter. Internal diameter of the hollow fibres or fibrous membranes may be of about 180-200 µm. More generally, the trap portion 8 can have a first end having several axial inlets 11a, possibly formed by the ends of the hollow fibrous guides 11, to allow a TFF separation (tangential flow filtration).
The channels, preferably arranged parallel along the longitudinal axis A at mounted state of the filtering assembly 1 having such device, are configured to allow for tangential flow filtration. Membrane/fibrous thickness for each of the guides 11 may be inferior to the internal diameter, such thickness being between, 20 and 55 µm, for instance of 30 or 40 µm.
Many fluid flow-paths, which are longitudinal, can be thus defined, each extending between the axial inlet 11a and the axial outlet 11b provided in the guide end, here formed at the filter insertion end. The filter outlet 1b

Since the plugging part has an identical or equivalent fixation area and is adapted to support the trap portion 8, the filter device can be an insertable filter unit of the preassembled type.
A mounting head 6 less narrow than the insertion body is obtained, whereby integration of this filter device 5 can be similar to the device according to first and second embodiments: Any interface CC, for instance a bag port connector/coupler CC (coupler C2, C2'), a tube-to-tube coupler 120, a coupler 20 for Tri-clamp^{®} system or similar clamping arrangement can be used with the filter device 5 housed in the tubular male member 4a: the insertion body is entirely received in the lumen 40.

As apparent in Fig. 10, the filter device 5 can be inserted either through the free end (distal end of the coupler) or an inner side corresponding to inner face F of the flange 30, for an interface CC carried by a bag 2 or similar fluid receiving device. In each case, the mounting head has an axial opening O6, possibly provided with a grid or mesh portion forming the filter inlet 1a.

In any of the options/variants to form the trap portion 8, 108, it is understood that the radial extension dr formed by the radial size of the lip 6c may be inferior or equal to 2 mm. More generally, the radial extension dr may be:
- at least twice lower than an opening diameter of the mounting head 6 and inferior or equal to 2 mm when the opening diameter is inferior or equal to 9.6 mm;
- at least three times or four times lower than an opening diameter of the mounting head 6 when the opening diameter is comprised between 9.6 mm and 25 mm.
This ensures that the mounting head 6 is not interfering with proper and quick mounting of a hose T, when coupled to a barb fitting or similar male connector for hose attachment.

### Fifth embodiment of a filter device, with a porous structure to form the trap portion

Referring to Figs 11 and 13A-13B, it shown an option where the filter device 5 has a trap portion 8 integrated in a piece forming the outer end (rear end) of the filter device 5, like in the first embodiment. Here, the trap portion 8 can entirely be made of monolith of porous material, possibly ceramic, polymer, metal. A mineral composition of the porous structure can be preferred, for instance with a monolithic silica structure or 3D printing of superposed mesh parts or beads/spheres. The monolith has a filter insertion end 7 that can form a front face F7 at the opposite from the rear end of the monolith provided at the outer end of the filter device 5, in the mounting head 6. A lip 6c may be provided adjacent to the groove G for the sealing element J, as reflected in Fig. 13A for instance. More generally, a porous structure PS is here used to form the trap portion 8, with the plugging part being axially permeable to the liquid to be filtered.

The overall shape and functions of the plugging part in the filter device, formed by or carrying the porous structure PS, corresponds substantially to those provided for the first embodiment of the filter device 5. Retention of the visible solid particles inside the filter device interior volume is efficiently obtained. Accordingly, reference is made in particular to differences with regard to the structure of the trap portion 8, here included in/having the porous structure PS, typically on a whole length L of the trap portion 8.
The porous structure PS, which is a rigid monolithic structure, has inner pores 81 distributed along and around the central axis X in the trap portion 8, so that such inner pores 81 are arranged close to the longitudinal axis A of the segment of conduit SC in mounted state. The inner pores 81 form an interconnected porous net for circulation of liquid entering in the filter device 5 via the inlet 1a of the mounting head 6. More generally, an inner circulation space PH can be formed by the pores (with sinuous circulation), with the pores preferably distributed regularly over the entire trap portion length L.

The porous structure PS can be a 3D structure elongated (longer than wide), produced by additive manufacture, molding or a known method. Optionally, the porous structure PS also has outer pores 82, in fluid communication with the inner pores 81, which are outer pores opening in the lumen 40. In such case, the trap portion 8 is permeable and configured to allow radial filtration in part, which is centrifugal, inside the lumen 40.
The size and geometry considerations given for other embodiments or in generic way may apply, of course, also for this fifth embodiment of the filter device 5 as for any of the options given to obtain a compact trap portion 8, 108 that extends longitudinally inside the segment of conduit SC.

### Exemplary securement to fix the filter device

The filtering assembly 1, 101 may include a securement mechanism to fix the filter device 5 in place within the coupler C2, C2', 20, 120 forming the fluid connection interface CC. The securement mechanism may engage internal features of the segment of conduit SC. As depicted, the securement mechanism is or includes a lip 6c or collar projecting outwardly from the plugging part, at the outer/rear end thereof. In some embodiments, the lip 6c or collar may abut an end of the tubular member 4a without any specific rotational position required.

The lip 6c, collar or similar retaining part may be received in an interior recess or groove of/defined by the segment of conduit SC (see recesses provided in Fig. 10 for instance). The securement mechanism allows the section conduit SC to prevent the filter device 5 from being flushed downstream when fluid is flowing through the segment of conduit SC.
More generally, a filtering assembly 1 or 101 can be obtained with the filter device 5 (for instance a compact device of any kind of the above described embodiments) remaining fixed in its rotational position and its axial position, thanks to the plugging part engagement with a fitting action exerted at the sealing .

### Options with a filter device included in a genderless aseptic connection system

A filter device 5 can be used to be coaxially arranged with a barb fitting/fluidic male member 4a and a hose T, possibly with the lumen 40 provided away from a flange 30 and/or provided in a tube-to tube connector. The flange 30 may be involved in a genderless aseptic connection. Instead of being included in bag port fitment attached to at least one film sheet of a bag unit (to close an opening of the bag unit), the male member 4a of the hollow fluid connection interface may be included in an adapter such as a Tri-clamp^{®} component. In such embodiment, the fluidic connection member 4 can define a central passage that combines the lumen 40 of the segment of conduit SC and an enlarged section pathway 38, typically arranged along the longitudinal axis A. The fluidic connection member 4 can taper from a base of the attachment body CB, toward a junction with the male member 4a. A hose barb connecting element/member, forming the male member 4a, is formed in the plastic or metal piece forming this component, with the lumen 40 formed in the section/segment of conduit SC, to be narrower than an inner diameter of the annular base of the Tri-clamp^{®} component (inner diameter of the enlarged section pathway).

In an embodiment, two jaws C1, C1' of a clamping member can be provided (Fig. 3A) to allow a coupling of the flange 30 of the attachment body with another/complementary flange provided in a device forming a mounting device. A three-part assembly TC (of the Tri-Clamp^{®} type) may be formed with the two jaws that can be in a closed configuration to clamp the flange 30 with the complementary flange 30'. While a hinge linking HL of the two jaws C1, C1' and a locking mechanism LM (at the opposite from the hinge) are shown in Fig. 3A, any other suitable coupling may be chosen to ensure a sealed connection for fluid transfer via the filtering assembly 101. One or more sealing parts of annular shape may be provided in these embodiments, in interposed position (front position on the flange) between the flanges of the genderless connectors.
The interface of each genderless connector or clamping connector may be arranged around an opening (axial/central opening) that is wider than a diameter of the lumen 40.

Of course, the filter device 5 can also be included in a aseptic male-female connection system, for instance using a bayonet connection, a spring valve connection or any connection involving a valve. One or more sealing parts of annular shape may be provided in these embodiments, in interposed position between an inserted fitting and a receiving fitting.

### Use in exemplary applications for delivery of purified product

A filter device 5 can be centrally mounted, being coaxially arranged with a barb fitting/fluidic connecting member 4 and a hose T. The male member 4a can have a smooth inner surface of annular section, typically circular, to allow an insertion from the free end 4e or the other side. The male member 4a can be the coupling part of the port fitment of a bag 2 for hose attachment or the coupling part of any line suitable for delivering/transferring the product (possible for final filling).

For instance, the filtering assembly 1, 101 is provided in a circuit or flowing path in communication with a dose forming apparatus that receives the filtered product, without any subsequent filtering downstream the filter device 5. The dose forming apparatus can be configured to deliver a dose made of the filtered product.
One or more final biopharmaceutical drug components, having molecules, biomedical compounds or cells of characteristic size or diameter inferior to 50 micrometres for instance, are included in the flowable biopharmaceutical product BF that is circulated through the filter device 5 in the circuit or flowing path.

The filter device is ready to be used, once integrated in filtering assembly 1, 101. In options for use with a bag unit, chosen amongst a parenteral product bag and a bioreactor, the filter device 5 can be directly inserted in the male tubular member 4, thus being placed between two sheets 2c of plastic material serving to form a wall W delimiting the interior volume V (sterile). A filtering assembly 1 is thus obtained, and can allow the biopharmaceutical product BF supplied from the bag 2, though the filter device 5, to be free/essentially free of visible particulate matter (≥ 100µm).

Besides, it is understood that any suitable structure can be provided to have a trap portion 8, 108 that is small, compact while being elongated in the overall geometry of the filter device 5, which may have its higher size (length) inferior to 30 or 40 mm.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A flowable biopharmaceutical product filtering assembly (1; 101), comprising:
- a hollow fluid connection interface (CC) having two opposite ends and allowing circulation of the product;
- a filter device (5) disposed within the hollow fluid connection interface, in an inserted position;
wherein the fluid connection interface (C2; C2'; 20; 120) includes a fluidic connection member (4) comprising a segment of conduit (SC) defining a lumen (40) through which the flowable biopharmaceutical product (BF) can pass, the segment of conduit (SC) having a longitudinal axis (A) and extending longitudinally:
- between a free end (4e) of a tubular male member (4a), which is an elongated connecting member configured for attachment to a flexible hose (T);
- and a mouth (4b), which is preferably an axial mouth,
wherein the filter device (5) is configured to selectively retain visible solid particles which have a size superior or equal to 100 micrometres, the filter device (5) comprising:
- a plugging part that is permeable;
- a filter insertion end (7); and
- a trap portion (8; 108) that is elongated, along the longitudinal axis (A), while being disposed adjacent to the plugging part and configured to purify the flowable biopharmaceutical product by separating and keeping the visible solid particles downstream the plugging part, between the plugging part and the filter insertion end (7) provided opposite from the plugging part;
and wherein the trap portion (8; 108) extends inside the lumen (40) and is axially locked or maintained in the inserted position by the plugging part.

2. The filtering assembly according to claim 1, wherein the plugging part has a central passage, preferably closed by a grid or permeable radial portion, the central passage extending from an open axial free end to a central access defining an inlet of the trap portion.

3. The filtering assembly according to claim 1 or 2, wherein the fluid connection device consists of a fitment (20) provided with an attachment body (3) comprising a flange (30) for attachment to an opening (O; O2) of a fluid containing device having an interior volume (V) for storage and/or circulation of the product, whereby the filter device (5) is disposed within the fitment (20) in the inserted position;
wherein the segment of conduit (SC) extends longitudinally:
- between the free end of a the tubular male member (4a) that is protruding from the attachment body (3) away from the interior volume (V),
- and said mouth (4b) where the lumen (40) is in fluid communication with and/or opens into the interior volume (V).

4. The filtering assembly according to claim 3, which is a filtering assembly (1) for a plastic bag (2) having a flexible wall (W) made of one or more flexible plastic sheets (2c) to delimit a closed and sterile chamber forming the interior volume (V), the filtering assembly (1) comprising:
- a bag opening extending through the flexible wall (W) and forming said opening (O; O2);
- the fitment (20) provided with the attachment body (3) in contact with and secured to said wall (W) at said opening (O; O2), the fitment (20) closing the opening (O; O2).

5. The filtering assembly according to claim 4, wherein the plugging part is a mounting head (6; 6') of the filtering device (5) in continuous annular contact with the segment of conduit (SC),
wherein the segment of conduit (SC) is integrally formed with the attachment body (3) that comprises:
- the flange (30) for attachment to one or two of the one or more plastic sheets (2c) of the plastic bag (2), the attachment body (3) having a base (B3) at the flange (30) with the base elongated along a given direction;
- an outer face (F3), and
- an inner face (F) arranged opposite the outer face (F3), with said mouth (4b) provided in the base (B3) and opening to the interior volume (V) through the inner face (F), so that the base (B3) is crossed by the lumen (40) that extends perpendicular to the given direction,
and wherein the tubular male member (4a) protrudes, along the longitudinal axis (A), from the outer face (F3).

6. The filtering assembly according to claim 5, wherein the mounting head (6; 6') has a fitting part (6f) that provides an annular sealing contact against a segment end of the segment of conduit (SC),
and wherein the mounting head (6), facing away from the inserting portion by a mounting head outer face provided outside the lumen (40), is:
- permeable to liquids and solids to be retained by the trap portion (8);
- provided with a maximal outer diameter or width of the filter device at an external part adjacent to the fitting part (6f), the maximal outer diameter or width being greater than any internal diameter of the tubular male member (4a), and preferably greater than a maximal diameter of the lumen.

7. The filtering assembly according to claim 5 or 6, comprising a wall portion, delimiting the opening (O; O2) and including at least one film, of the plastic bag (2) that is preferably a single-use bag,
wherein the attachment body (3) and the tubular male member (4a) are part of an outlet in the fitment (20) carried by the plastic bag (2),
and wherein the mounting head (6; 6') of the filtering device (5) axially presses onto the inner face (F) to maintain the trap portion (8) in a predefined inserted state.

8. The filtering assembly according to claim 5 or 6,comprising a wall portion, delimiting the opening (O; O2) and including at least one film, of the plastic bag (2) that is preferably a single-use bag,
wherein the attachment body (3) and the tubular male member (4) are part of an inlet in the fitment (20) carried by the plastic bag (2),
and wherein the mounting head (6; 6') of the filtering device (5) axially presses onto an annular end of the tubular male member (4a) to maintain of the trap portion (8) in a predefined inserted state.

9. The filtering assembly according to any one of the preceding claims, wherein the trap portion (8) has a maximum diameter (D8) lower than 14 or 19 mm and is longer than wide with a length (L) of the trap portion at least twice superior to said maximum diameter (D8).

10. The filtering assembly according to any one of the preceding claims, wherein the plugging part is at least 3 mm wider than the trap portion (8) or a central hollow (C4) thereof, while having a maximum outer diameter (D6) or width inferior to 30 mm.

11. The filtering assembly according to any one of th/.e preceding claims, wherein the plugging part includes a fluid filter inlet (1a) of the filtering assembly (1; 101), the fluid inlet (1a) communicating with a central hollow (C4) or with a porous structure (PS) of the trap portion (8),
and wherein the fluid filter inlet (1a) is an axial inlet, while a side wall of the trap portion (8) is permeable and configured to allow radial filtration, which is centrifugal, inside the lumen.

12. The filtering assembly according to any one of the claims 1-11, wherein the filter device (5) has the insertion filter end (7) and the plugging part constructed together as a monolithic part or included in a pre-assembled unit assembled before being mounted in the lumen (40).

13. The filtering assembly according to any one of the claims 1-11, wherein the trap portion (8) has a tapering profile and at least one filtering membrane or media extending longitudinally with two elongated sides of the membrane or media fixed to two triangular flanges or sides of the rigid trap portion.

14. The filtering assembly according to any one of the claims 1-11, wherein the trap portion (8) is made of a component provided with a tubular mesh or a 3D porous structure that is rigid and performing retention of the visible solid particles inside a filter device interior volume.

15. The filtering assembly according to any one of the claims 1-11, wherein the plugging part is provided with a fitting part (6f) that provides an annular sealing contact against a segment end of the segment of conduit (SC), so that the plugging part is partially inserted inside the tubular male member (4a), away from the mouth (4b),
and wherein the plugging part, facing outside the lumen (40) by a mounting head outer face (F6), is closing a cavity for a filling of granular material (B), the granular material (B) forming all or part of the trap portion (8).

16. The filtering assembly according to any one of the claims 1-11, wherein the trap portion (8) comprises channels formed by hollow guides having fibres, with the channels preferably arranged parallel along the longitudinal axis (A) for tangential flow filtration.

17. The filtering assembly according to any one of the claims 1-11, wherein the trap portion (8) comprises a porous rigid monolithic structure (PS) with inner pores (81) distributed along and around the longitudinal axis (A) in the trap portion, the structure also having outer pores (82), in fluid communication with the inner pores (81), which are outer pores opening in the lumen (40).

18. The filtering assembly according to any one of the claims 1-3, wherein the fluid connection device is one amongst a tube-to-tube connector and a single piece coupler of a tube-to-tube connection system,
and wherein the lumen (40) extends rectilinearly along the longitudinal axis (A) and opens into a chamber for fluid circulation.
Optionally, the mouth (4b) provided at a section transition region is narrowed, possibly progressively narrowed, toward the segment of conduit (SC).

19. Use of the filtering assembly (1; 101) according to any one of the preceding claims,
wherein the filtering assembly is provided in a circuit or flowing path,
wherein one or more final biopharmaceutical drug components, having molecules, biomedical compounds or cells of characteristic size or diameter inferior to 50 micrometres, are included in the flowable biopharmaceutical product (BF) that is circulated through the filter device (5) in the circuit or flowing path to be filtered,
and wherein the filtered product reaches then a dose forming apparatus, without any subsequent filtering downstream the filter device (5), the dose forming apparatus being configured to deliver a dose made of the filtered product.

20. A method for assembling a bag unit, chosen amongst a single-use bag and a single-use bioreactor, with integration of a filter device (5) through a lumen (40) in a male tubular member (4a), the bag unit including sheets (2c) of plastic material to form a wall (W) delimiting a sterile interior volume (V), the method comprising:
- providing a fitment (C2; C2') forming a hollow fluid connection interface, through an opening (O; O2) of the wall (W), the fitment (C2; C2') including a fluidic connection member (4) provided with the male tubular member (4a) ;
- inserting a filter device (5) inside the lumen (40), in an inserted position, with the filter device (5) having a plugging part, a trap portion (8) configured to selectively retain visible solid particles which have a size superior or equal to 100 micrometres downstream the plugging part, between the plugging part and a filter insertion end (7) provided opposite from the plugging part in the filter device (5); and
- maintaining the inserted position with the trap portion (8) disposed adjacent to the plugging part, inside the lumen (40), by an axially locking action performed by the plugging part against a segment of conduit (SC) of the male tubular member (4a) where the lumen (40) is delimited, in order to obtain the flowable biopharmaceutical product filtering assembly (1) according to any one of claims 1-18.
